(19) Europäisches Patentamt
European Patent Office
Office européen des brevets

(11) EP 4 489 552 A1

(12) **EUROPEAN PATENT APPLICATION**
published in accordance with Art. 153(4) EPC

(43) Date of publication:
**08.01.2025 Bulletin 2025/02**

(21) Application number: **23763528.9**

(22) Date of filing: **02.03.2023**

(51) International Patent Classification (IPC):
***H10K 85/60*** *(2023.01)* ***C09K 11/06*** *(2006.01)*
***H10K 50/12*** *(2023.01)* ***H10K 71/70*** *(2023.01)*
***H10K 101/20*** *(2023.01)* ***H10K 101/30*** *(2023.01)*

(52) Cooperative Patent Classification (CPC):
**C09K 11/06; H05B 33/10; H10K 50/10; H10K 50/12; H10K 71/70; H10K 85/60;**
H10K 2101/20; H10K 2101/30

(86) International application number:
**PCT/JP2023/007738**

(87) International publication number:
**WO 2023/167271 (07.09.2023 Gazette 2023/36)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.03.2022 JP 2022033872**

(71) Applicant: **Kyulux, Inc.**
**Fukuoka-shi, Fukuoka 819-0388 (JP)**

(72) Inventors:
- **KAKIZOE, Hayato**
  **Fukuoka-shi, Fukuoka 819-0388 (JP)**
- **YOSHIZAKI, Asuka**
  **Fukuoka-shi, Fukuoka 819-0388 (JP)**

(74) Representative: **Zimmermann & Partner Patentanwälte mbB**
**Postfach 330 920**
**80069 München (DE)**

(54) **ORGANIC LIGHT-EMITTING ELEMENT, METHOD FOR EVALUATING DELAYED FLUORESCENCE MATERIAL, METHOD FOR DESIGNING DELAYED FLUORESCENCE MATERIAL, METHOD FOR DESIGNING ORGANIC LIGHT-EMITTING ELEMENT, AND PROGRAM**

(57) An organic light emitting device having a light emitting layer that contains a delayed fluorescent material having a difference between PBHT(Tn) and PBHT(T1) of 0.01 or more has excellent durability. PBHT(T1) represents the PBHT of the lowest excited triplet state of a delayed fluorescent material, and PBHT(Tn) represents the PBHT of an excited triplet state having the smallest energy among excited triplet states having larger energies than the lowest excited singlet energy of the delayed fluorescent material.

FIG. 1

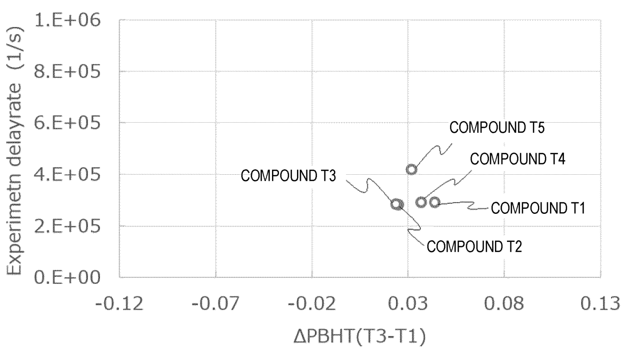


EP 4 489 552 A1

Processed by Luminess, 75001 PARIS (FR)

## Description

### Technical Field

The present invention relates to an organic light emitting device having high durability, a method for designing the organic light emitting device, a method for evaluating a delayed fluorescent material that is useful as a material for the organic light emitting device, a method for designing the delayed fluorescent material, and a program for carrying out these methods.

### Background Art

Studies for enhancing the light emission efficiency of organic light emitting devices such as organic electroluminescent devices (organic EL devices) are being conducted actively. In particular, various kinds of efforts have been made to increase the light emission efficiency of organic light-emitting devices by newly developing the materials constituting the devices. Among them, there are seen some studies related to organic electroluminescent devices that utilize a delayed fluorescent material as the material of a light emitting layer.

A delayed fluorescent material is a compound which, in an excited state, undergoes reverse intersystem crossing from an excited triplet state to an excited singlet state and then emits fluorescence when returning back from the excited singlet state to a ground state thereof. Fluorescence emitted through such a route is observed later than fluorescence from the excited singlet state directly occurring from the ground state (ordinary fluorescence), and is therefore referred to as delayed fluorescence. Here, for example, in a case where an organic molecule is excited through carrier injection, the probability of occurrence of the excited singlet state to that of the excited triplet state is statistically 25% : 75%. Therefore, in conventional fluorescent materials that utilize only the energy of the excited singlet state occurring directly from the ground state, there is a limitation in the improvement of light emission efficiency. On the other hand, in delayed fluorescent materials, not only the excited singlet state but also the excited triplet state can be utilized for fluorescent emission through a route involving the above-mentioned reverse intersystem crossing, and therefore, high light emission efficiency is obtained as compared with an ordinary fluorescent material.

Incidentally, it is believed that the reverse intersystem crossing from triplet to singlet such as described above is more likely to occur as the difference $\Delta E_{ST}$ between the lowest excited singlet energy and the lowest excited triplet energy is smaller. Meanwhile, $\Delta E_{ST}$ tends to exhibit a smaller value as the overlap between the HOMO orbital and the LUMO orbital is smaller. Therefore, in a compound in which the overlap between the HOMO orbital and the LUMO orbital is smaller, the $\Delta E_{ST}$ becomes smaller, and the reverse intersystem crossing is likely to occur. From these, in conventional research and development of delayed fluorescent materials, molecular design has been carried out with the aim of, for example, making the $\Delta E_{ST}$ less than 0.2, or bringing the index values that indicate the degree of overlap of the HOMO orbital and the LUMO orbital (PBHT value, see NPL 1) closer to each other. The PBHT value has a value of 0 or more and 1 or less and is a calculated value indicating that as the PBHT value is closer to 0, the degree of orbital overlap is smaller.

### Citation List

### Patent Literature

NPL 1: J. Chem. Phys. 128, 044118 (2008)

### Summary of Invention

### Technical Problem

As described above, until now, the molecular design of delayed fluorescent materials has been carried out using the $\Delta E_{ST}$ and the PBHT value as indices of the likelihood of reverse intersystem crossing. However, when the inventors of the present invention investigated the relationship between the $\Delta E_{ST}$ or PBHT value and the delayed fluorescence rate, it was found that materials having a small $\Delta E_{ST}$ or a PBHT value close to 0 certainly tend to have a fast delayed fluorescence rate; however, among them, there are some materials that do not exhibit a sufficient delayed fluorescence rate, and that when such a delayed fluorescent material is used in a light emitting layer, the durability of the device is decreased due to long-lived triplet excitons.

Thus, the present inventors conducted extensive investigations for the purpose of providing an organic light emitting device that contains a delayed fluorescent material in a light emitting layer and has excellent durability.

Solution to Problem

As a result of conducting intensive investigations, the present inventors have found that among delayed fluorescent materials, a group of compounds in which the PBHT value of higher excited triplet state Tn is larger than the PBHT value of the lowest excited triplet state T1, tends to have a fast delayed fluorescence rate. Then, the inventors found that an organic light emitting device having excellent durability can be realized by using such a delayed fluorescent material in the light emitting layer.

The invention has been proposed based on these findings and specifically has the following configurations.

[1] An organic light emitting device including a light emitting layer containing a delayed fluorescent material having a ΔPBHT of 0.01 or more as defined by the following formula (I):

$$\text{Formula (I)} \qquad \Delta PBHT = PBHT(Tn) - PBHT(T1)$$

in which in formula (I), PBHT(T1) represents a PBHT value of a lowest excited triplet state of the delayed fluorescent material, and PBHT(Tn) represents a PBHT value of an excited triplet state having a smallest energy among excited triplet states having larger energies than a lowest excited singlet energy of the delayed fluorescent material.

[2] The organic light emitting device according to [1], in which the delayed fluorescent material is a compound represented by any of the following general formulae (1) to (6):

General Formula (1)

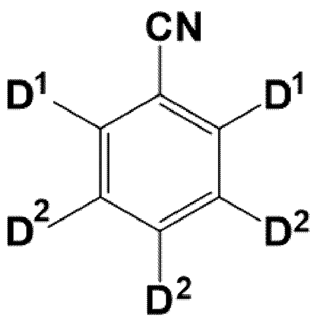

General Formula (2)

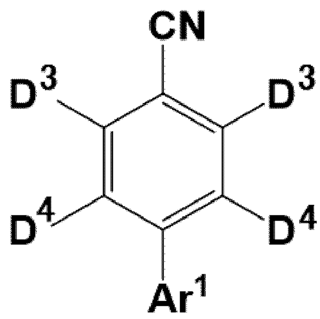

General Formula (3)

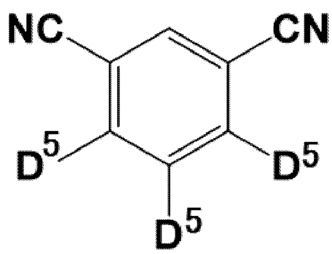

General Formula (4)

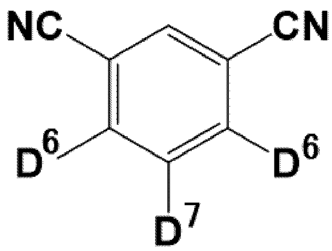

General Formula (5)

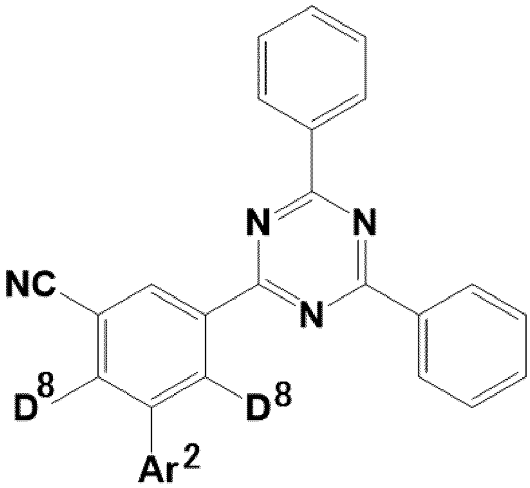

General Formula (6)

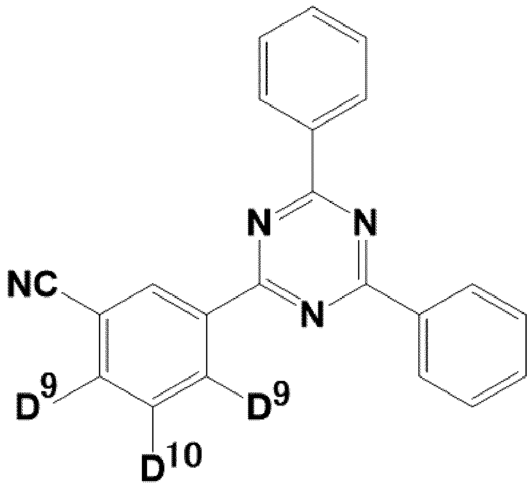

in which in general formulae (1) to (6), $D^1$ to $D^{10}$ each independently represent a group represented by the following general formula (7), provided that $D^1$ and $D^2$, $D^3$ and $D^4$, $D^6$ and $D^7$, and $D^9$ and $D^{10}$ have chemical structures different from each other, and two $D^1$'s, three $D^2$'s, two $D^3$'s, two $D^4$'s, three $D^5$'s, two $D^6$'s, two $D^8$'s, and two $D^9$'s have chemical structures identical with each other;

General Formula (7)

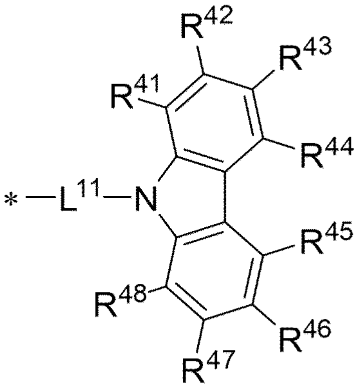

in which in general formula (7), $L^{11}$ represents a single bond or a divalent linking group; $R^{41}$ to $R^{48}$ each independently represent a hydrogen atom or a substituent; $R^{41}$ and $R^{42}$, $R^{42}$ and $R^{43}$, $R^{43}$ and $R^{44}$, $R^{44}$ and $R^{45}$, $R^{45}$ and $R^{46}$, $R^{46}$ and $R^{47}$, and $R^{47}$ and $R^{48}$ may be bonded to each other to form a cyclic structure.

[3] The organic light emitting device according to [2], in which in each of the general formulae (1) to (6), at least one of the groups represented by the general formula (7) is a group represented by any of the following general formulae (8) to (13):

General Formula (8)

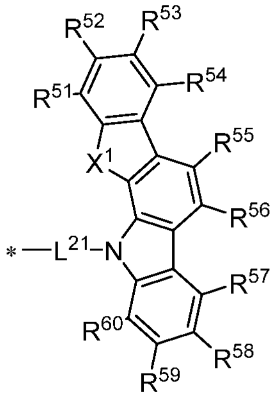

General Formula (9)

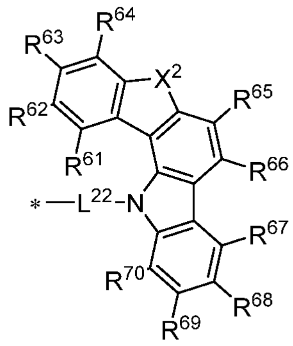

General Formula (10)

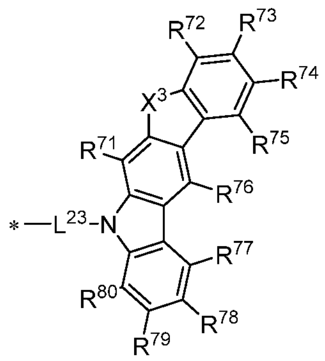

General Formula (11)

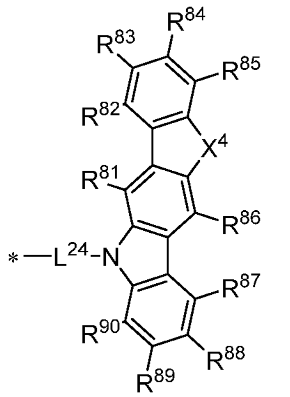

General Formula (12)

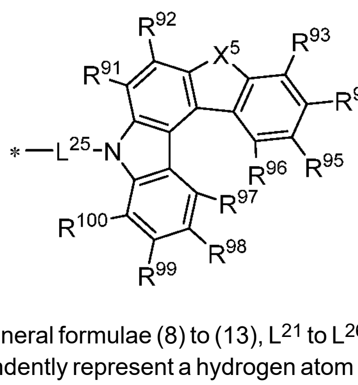

General Formula (13)

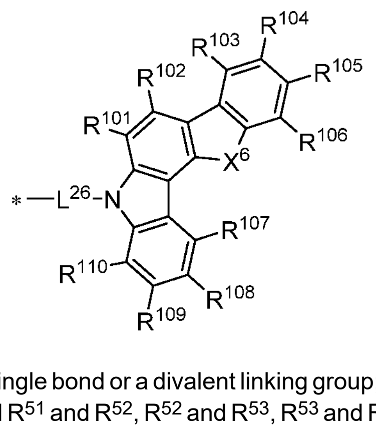

in which in general formulae (8) to (13), $L^{21}$ to $L^{26}$ each represent a single bond or a divalent linking group; $R^{51}$ to $R^{110}$ each independently represent a hydrogen atom or a substituent; and $R^{51}$ and $R^{52}$, $R^{52}$ and $R^{53}$, $R^{53}$ and $R^{54}$, $R^{54}$ and $R^{55}$, $R^{55}$ and $R^{56}$, $R^{56}$ and $R^{57}$, $R^{57}$ and $R^{58}$, $R^{58}$ and $R^{59}$, $R^{59}$ and $R^{60}$, $R^{61}$ and $R^{62}$, $R^{62}$ and $R^{63}$, $R^{63}$ and $R^{64}$, $R^{65}$ and $R^{66}$, $R^{66}$ and $R^{67}$, $R^{67}$ and $R^{68}$, $R^{68}$ and $R^{69}$, $R^{69}$ and $R^{70}$, $R^{72}$ and $R^{73}$, $R^{73}$ and $R^{74}$, $R^{74}$ and $R^{75}$, $R^{75}$ and $R^{76}$, $R^{76}$ and $R^{77}$, $R^{77}$ and $R^{78}$, $R^{78}$ and $R^{79}$, $R^{79}$ and $R^{80}$, $R^{81}$ and $R^{82}$, $R^{82}$ and $R^{83}$, $R^{83}$ and $R^{84}$, $R^{84}$ and $R^{85}$, $R^{86}$ and $R^{87}$, $R^{87}$ and $R^{88}$, $R^{88}$ and $R^{89}$, $R^{89}$ and $R^{90}$, $R^{91}$ and $R^{92}$, $R^{93}$ and $R^{94}$, $R^{94}$ and $R^{95}$, $R^{95}$ and $R^{96}$, $R^{96}$ and $R^{97}$, $R^{97}$ and $R^{98}$, $R^{99}$ and

$R^{100}$, $R^{101}$ and $R^{102}$, $R^{102}$ and $R^{103}$, $R^{103}$ and $R^{104}$, $R^{104}$ and $R^{105}$, $R^{105}$ and $R^{106}$, $R^{107}$ and $R^{108}$, $R^{108}$ and $R^{109}$, and $R^{109}$ and $R^{110}$ may be bonded to each other to form a cyclic structure.

[4] The organic light emitting device according to any one of [1] to [3], in which the PBHT(Tn) is PBHT(T3) of a third excited triplet state.

[5] The organic light emitting device according to [4], in which the delayed fluorescent material is a compound represented by the general formula (2).

[6] The organic light emitting device according to [5], in which $D^4$ in the general formula (2) is a group represented by the general formula (13).

[7] The organic light emitting device according to any one of [1] to [3], in which the PBHT(Tn) is PBHT(T2) of a second excited triplet state.

[8] The organic light emitting device according to [7], in which the delayed fluorescent material is a compound represented by the general formula (4) or (5).

[9] The organic light emitting device according to [8], in which $D^6$ in the general formula (4) and $D^8$ in the general formula (5) are each a group represented by the general formula (13).

[10] A method for evaluating a delayed fluorescent material, the method including:
evaluating light emission characteristics of a delayed fluorescent material based on ΔPBHT defined by the formula (I).

[11] The evaluation method according to [10], including:
predicting a delayed fluorescence rate of the delayed fluorescent material based on the ΔPBHT.

[12] The evaluation method according to [10] or [11], further including:

determining a relationship between ΔPBHT and a delayed fluorescence rate $\tau 2$ based on ΔPBHT values and delayed fluorescence rates of a plurality of kinds of reference delayed fluorescent materials having different ΔPBHT values;
calculating ΔPBHT of a delayed fluorescent material as a target for evaluation, determining a value of the delayed fluorescence rate corresponding to the ΔPBHT of the target for evaluation from the relationship between the ΔPBHT and the delayed fluorescence rate $\tau 2$, and predicting this value to be the delayed fluorescence rate of the target for evaluation; and
evaluating light emission characteristics of the target for evaluation based on the predicted delayed fluorescence rate.

[13] The evaluation method according to [12], in which the delayed fluorescence rates of the reference delayed fluorescent materials are measured values.

[14] A method for designing a delayed fluorescent material, the method including:
performing molecular design of a delayed fluorescent material based on a relationship between a structure of the delayed fluorescent material and ΔPBHT defined by the formula (I).

[15] The designing method according to [14], including:

a first step of calculating ΔPBHT of a specific delayed fluorescent material;
a second step of designing a modified compound in which a part of a structure of the specific delayed fluorescent material is changed, and calculating ΔPBHT of the modified compound;
a third step of designing a remodified compound in which a part of a structure of the modified compound is changed, and calculating ΔPBHT of the remodified compound;
a fourth step of determining a relationship between a compound structure and ΔPBHT based on structures of the specific delayed fluorescent material, the modified compound, and the remodified compound and the calculated ΔPBHT values; and
a fifth step of extracting a compound structure corresponding to ΔPBHT in a target range from the relationship between a compound structure and ΔPBHT, and selecting a delayed fluorescent material to be synthesized from a group of compounds having the extracted structure.

[16] The designing method according to [15], in which a partial change in the structures of the specific delayed fluorescent material and the modified compound is a quantifiable change.

[17] The designing method according to [15] or [16], further including:

determining a difference $\Delta E_{ST}$ between a lowest excited singlet energy and a lowest excited triplet energy for the specific delayed fluorescent material, the modified compound, and the remodified compound,
in which in the fifth step, a delayed fluorescent material to be synthesized is selected from the group of compounds, based on the $\Delta E_{ST}$ determined in the above-described step.

[18] The designing method according to any one of [15] to [17], in which the remodified compound designed in the third step is regarded as a modified compound, and the third step is repeatedly carried out.

[19] A method for designing an organic light emitting device, the method including:

selecting a delayed fluorescent material based on ΔPBHT defined by the formula (I), and designing an organic light emitting device using the selected delayed fluorescent material.

[20] The designing method according to [19], including:

- searching for a delayed fluorescent material having a ΔPBHT of 0.01 or more from a database of delayed fluorescent materials storing ΔPBHT values of a plurality of kinds of delayed fluorescent materials as data;
- selecting a delayed fluorescent material to be used in an organic light emitting device, from a group of delayed fluorescent materials found in the search in the above-described step; and
- designing an organic light emitting device using the delayed fluorescent material selected in the above-described step.

[21] The designing method according to [20], in which the database of delayed fluorescent materials further stores differences $\Delta E_{ST}$ between a lowest excited singlet energy and a lowest excited triplet energy of the plurality of delayed fluorescent materials as data, and in the step of searching for a delayed fluorescent material, a delayed fluorescent material having a ΔPBHT of 0.01 or more and a $\Delta E_{ST}$ of 0.3 eV or less is searched for from the database of delayed fluorescent materials.

[22] A program for carrying out the method according to any one of [10] to [21].

## Advantageous Effects of Invention

According to the present invention, an organic light emitting device having excellent durability can be realized by using a delayed fluorescent material having a ΔPBHT of 0.01 or more. Furthermore, according to the method for evaluating a delayed fluorescent material of the invention, the light emission characteristics of a delayed fluorescent material, such as the delayed fluorescence rate, can be easily evaluated. In addition, according to the method for designing a delayed fluorescent material of the invention, a delayed fluorescent material having a high delayed fluorescence rate can be designed.

## Brief Description of Drawings

[FIG. 1] FIG. 1 is a graph showing the relationship between ΔPBHT(T3-T1) and the delayed fluorescence rate.
[FIG. 2] FIG. 2 is a graph showing the relationship between ΔPBHT(T2-T1) and the delayed fluorescence rate.
[FIG. 3] FIG. 3 is a graph showing the relationship between ΔPBHT(T3-T1) and LT95.
[FIG. 4] FIG. 4 is a graph showing the relationship between ΔPBHT(T2-T1) and LT95.
[FIG. 5] FIG. 5 is a graph showing the relationship between ΔPBHT(T2-T1) and the delayed fluorescence rate.
[FIG. 6] FIG. 6 is a graph showing the relationship between ΔPBHT(T2-T1) and LT95.
[FIG. 7] FIG. 7 is a flowchart showing a method for designing a delayed fluorescent material.

## Description of Embodiments

The contents of the invention will be described in detail below. Explanation of the configuration requirements described below may be based on representative embodiments and specific examples of the invention; however, the invention is not intended to be limited to such embodiments and specific examples. A numerical value range expressed herein using the term "to" means a range that includes the numerical values described before and after the term "to" as the lower limit value and the upper limit value. The term "consisting of" as used herein means consisting of only those items described after the term "consisting of", and does not include anything else. Some or all of hydrogen atoms present in the molecule of a compound used in the present invention can be substituted with deuterium atoms ($^2$H, deuterium D). In the chemical structural formulae in the present specification, a hydrogen atom is expressed as H, or expression thereof is omitted. For example, when expression of an atom bonded to a ring skeleton-constituting carbon atom of a benzene ring is omitted, it is considered that H is bonded to the ring skeleton-constituting carbon atom at the site where the expression is omitted. The term "substituent" as used in the present specification means an atom or an atomic group other than a hydrogen atom and a deuterium atom. On the other hand, the expressions "substituted or unsubstituted" and "may be substituted" mean that a hydrogen atom may be substituted with a deuterium atom or a substituted. Furthermore, the term "transparent" as used herein means that the transmittance to visible light is 50% or higher, preferably 80% or higher, more

preferably 90% or higher, and even more preferably 99% or higher. The transmittance to visible light can be measured using an ultraviolet and visible spectrophotometer.

<Organic Light Emitting Device>

An organic light emitting device of the invention has a light emitting layer containing a delayed fluorescent material having a ΔPBHT of 0.01 or more as defined by the following formula (I):

$$\text{Formula (I)} \qquad \Delta PBHT = PBHT(Tn) - PBHT(T1)$$

In formula (I), PBHT(T1) represents the PBHT value of the lowest excited triplet state of a delayed fluorescent material, and PBHT(Tn) represents the PBHT value of an excited triplet state having the smallest energy among excited triplet states having larger energy than the lowest excited singlet energy of the delayed fluorescent material. In the following description, the "excited triplet state having the smallest energy among excited triplet states having larger energy than the lowest excited singlet energy" in the invention may be collectively referred to as "higher excited triplet state Tn".

For example, when a delayed fluorescent material has a lowest excited triplet state T1, a second excited triplet state T2, and a third excited triplet state T3 as the excited triplet states, and the energy Esi of the lowest excited singlet state, the energy $E_{T1}$ of the lowest excited triplet state, the energy $E_{T2}$ of the second excited triplet state, and the energy $E_{T3}$ of the third excited triplet state satisfy a relationship shown by the following formula:

$$E_{T1} < E_{S1} < E_{T2} < E_{T3},$$

PBHT(T2), which is the PBHT value of the second excited triplet state T2, corresponds to PBHT(Tn), and ΔPBHT is determined by the following formula.

$$\Delta PBHT = PBHT(T2) - PBHT(T1)$$

Furthermore, when the energy Esi of the lowest excited singlet state, the energy $E_{T1}$ of the lowest excited triplet state, the energy $E_{T2}$ of the second excited triplet state, and the energy $E_{T3}$ of the third excited triplet state satisfy a relationship shown by the following formula:

$$E_{T1} < E_{T2} < E_{S1} < E_{T3},$$

PBHT(T3), which is the PBHT value of the third excited triplet state T3, corresponds to PBHT(Tn), and ΔPBHT is determined by the following formula.

$$\Delta PBHT = PBHT(T3) - PBHT(T1)$$

Here, the "lowest excited singlet energy" and the energy of the "lowest excited triplet state" according to the invention are the lowest excited singlet energy ($E_{S1}$) and the lowest excited triplet energy ($E_{T1}$) determined by the procedure described below. The energies of higher excited triplet states Tn such as the second excited triplet state T2 and the third excited triplet state T3 are values determined by molecular orbital calculation using quantum chemical calculation B3LYP/6-31G.

The "PBHT value" according to the invention is a value proposed by Michael J. Peach, Peter Benfield, Trygve Helgaker, and David J. Tozer, and is a value named after the initials of the four surnames. The PBHT value is a numerical value that represents the orbital properties of an excited state. There are singlet and triplet PBHT values; however, in the present invention, the triplet PBHT value is employed. A small PBHT value indicates that the excited state is charge-transfer (CT)-related, and a large PBHT value indicates that the excited state is localized electron (LE)-related. The PBHT value is a value A calculated by the following formula.

$$\Lambda = \frac{\sum_{i,a} \kappa_{ia}^2 \, O_{ia}}{\sum_{i,a} \kappa_{ia}^2}$$

The definition of each term in the above formula is as follows.

$$O_{ia} = \langle|\varphi_i||\varphi_a|\rangle = \int |\varphi_i(r)||\varphi_a(r)|\, dr$$

$\varphi_i$: occupied orbital
$\varphi_a$: virtual orbital

$$\begin{pmatrix} A & B \\ B & A \end{pmatrix}\begin{pmatrix} X \\ Y \end{pmatrix} = \omega \begin{pmatrix} 1 & 0 \\ 0 & -1 \end{pmatrix}\begin{pmatrix} X \\ Y \end{pmatrix}$$

$$\kappa_{ia} = X_{ia} + Y_{ia}$$

The calculation method for the PBHT value is described in detail in J. Chem. Phys., 128, 044118 (2008) "Excitation energies in density functional theory: An evaluation and a diagnostic test", the entire contents of which are incorporated herein by reference.

The PBHT value indicates the degree of overlap between the highest occupied molecular orbital (HOMO) and the lowest unoccupied molecular orbital (LUMO) and takes a value of 0 or greater and 1 or less. A PBHT value of 0 indicates that there is no overlap between the HOMO and the LUMO, and a PBHT value of 1 indicates that the HOMO and the LUMO overlap completely.

The organic light emitting device of the invention exhibits excellent durability by containing a delayed fluorescent material having a ΔPBHT, which is defined as described above, of 0.01 or more in the light emitting layer. This is speculated to be due to the following reason.

That is, a ΔPBHT of 0.01 or more means that the degree of overlap between the HOMO and the LUMO in the higher excited triplet state Tn is greater than that in the lowest excited triplet state T1, indicating that the molecular orbital distribution is significantly different between T1 and Tn. Therefore, it can be speculated that in a delayed fluorescent material having a ΔPBHT of 0.01 or more, the lowest excited triplet state T1 is charge transfer-related (CT-related), while the higher excited triplet state Tn is localized electron-related (LE-related). In such a delayed fluorescent material, it is believed that after transition from the lowest excited triplet state T1 to the higher excited triplet state Tn, the spin-orbit interaction works effectively to cause triplet-singlet spin conversion to occur easily, and a high delayed fluorescence rate (reverse intersystem crossing rate) is exhibited. Alternatively, effective spin conversion occurs similarly even when T1 is LE-related whereas Tn is CT-related. Therefore, it is speculated that in an organic light emitting device containing a delayed fluorescent material having a ΔPBHT of 0.01 or more in the light emitting layer, deterioration caused by long-lived triplet excitons is suppressed and excellent durability is exhibited.

A delayed fluorescent material having a ΔPBHT of 0.01 or more, which can be used in the invention, will be described in detail below.

[Delayed Fluorescent Material]

The "delayed fluorescent material" according to the invention is an organic compound that undergoes reverse intersystem crossing from an excited triplet state to an excited singlet state in an excited state, and emanates fluorescence (delayed fluorescence) when returning from the excited singlet state to the ground state. In the present invention, a material in which when the emission lifetime is measured using a fluorescence lifetime measurement system (such as a streak camera system manufactured by HAMAMATSU PHOTONICS K.K.), fluorescence with an emission lifetime of 100 ns (nanoseconds) or more is observed, is referred to as a delayed fluorescent material.

In the present invention, among such delayed fluorescent materials, a material having a ΔPBHT of 0.01 or more as defined by the above-described formula (I) is used. The ΔPBHT is preferably 0.02 or more, more preferably 0.03 or more, and may be selected from the range of, for example, 0.04 or more, may be selected from the range of 0.05 or more, may be selected from the range of 0.06 or more, or may be selected from the range of 0.07 or more.

With regard to the delayed fluorescent material having a ΔPBHT of 0.01 or more that is used in the invention, it is preferable that the difference $\Delta E_{ST}$ between the lowest excited singlet energy and the lowest excited triplet energy at 77 K is 0.3 eV or less, more preferably 0.25 eV or less, more preferably 0.2 eV or less, more preferably 0.15 eV or less, even more preferably 0.1 eV or less, still more preferably 0.07 eV or less, even more preferably 0.05 eV or less, still more preferably 0.03 eV or less, and particularly preferably 0.01 eV or less.

It is preferable that the delayed fluorescent material having a ΔPBHT of 0.01 or more does not contain a metal atom. For example, as the delayed fluorescent material having a ΔPBHT of 0.01 or more, a compound consisting of atoms selected from the group consisting of a carbon atom, a hydrogen atom, a nitrogen atom, an oxygen atom, and a sulfur atom can be selected. For example, as the delayed fluorescent material having a ΔPBHT of 0.01 or more, a compound consisting of atoms selected from the group consisting of a carbon atom, a hydrogen atom, a nitrogen atom, and an oxygen atom can be selected. For example, as the delayed fluorescent material having a ΔPBHT of 0.01 or more, a compound consisting of a carbon atom, a hydrogen atom, and a nitrogen atom can be selected.

The delayed fluorescent material having a ΔPBHT of 0.01 or more can be selected from, for example, a group of compounds having a structure in which an aromatic hydrocarbon is substituted with one or more electron-withdrawing groups and one or more electron-donating groups. The aromatic ring constituting the aromatic hydrocarbon may be a monocyclic ring or a fused ring in which two or more rings are fused. A preferred example of the aromatic ring may be a benzene ring. The electron-withdrawing group is a group having a property of withdrawing electrons from the ring to which the electron-withdrawing group is bonded, and can be selected from, for example, groups having a positive Hammett σp value. The electron-donating group is a group having a property of donating electrons to the ring to which the electron-donating group is bonded, and can be selected from, for example, groups having a negative Hammett σp value.

Here, the "Hammett σp value" is a value proposed by L.P. Hammett to quantify the effect of substituents on the reaction rate or equilibrium of para-substituted benzene derivatives. For the explanation of the "Hammett σp value" and the numerical value of each substituent in the present invention, reference can be made to the description on the σp value in Hansch, C. et. al., Chem. Rev., 91, 165-195 (1991).

Examples of an acceptor group include a cyano group and a six-membered aromatic heterocyclic group containing a nitrogen atom as a ring skeleton-constituting atom. Examples of the 6-membered aromatic heterocyclic group containing a nitrogen atom as a ring skeleton-constituting atom include a substituted or unsubstituted pyridyl group, a substituted or unsubstituted pyrimidyl group, a substituted or unsubstituted pyridazyl group, a substituted or unsubstituted pyrazyl group, and a substituted or unsubstituted triazyl group. Examples of a donor group include a substituted or unsubstituted diphenylamino group and a group represented by general formula (7) that will be described below.

Particularly preferred examples of the delayed fluorescent material that can be used in the invention include delayed fluorescent materials which are compounds represented by any of the following general formulae (1) to (6) and have a ΔPBHT of 0.01 or more.

General Formula (1)

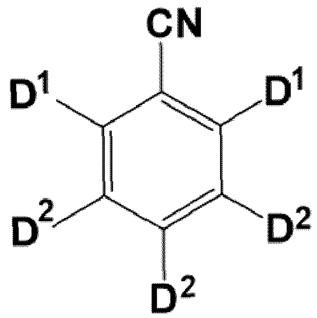

General Formula (2)

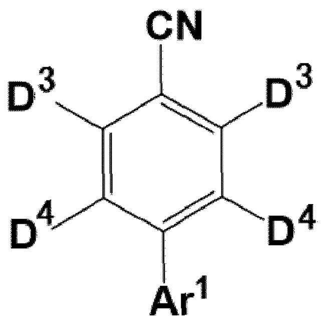

General Formula (3)

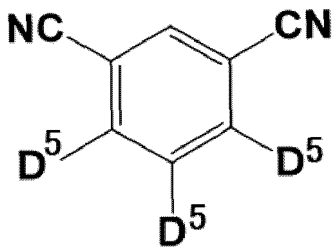

General Formula (4)

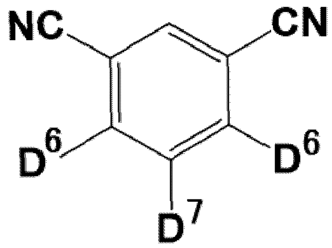

General Formula (5)

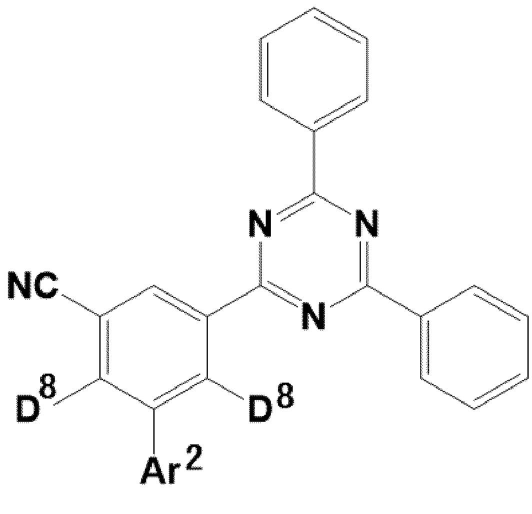

General Formula (6)

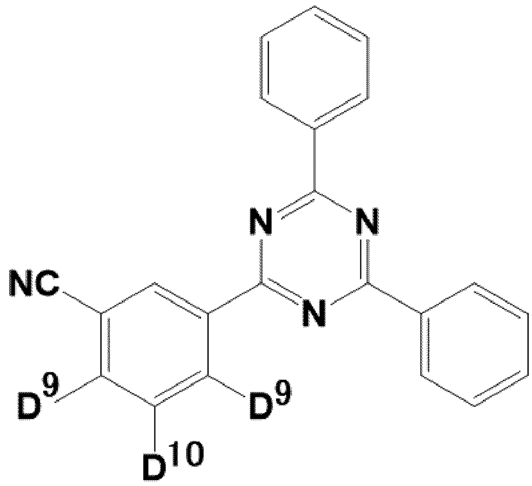

In general formulae (1) to (6), $D^1$ to $D^{10}$ each independently represent a group represented by the following general formula (7). However, each of the combinations of $D^1$ and $D^2$, $D^3$ and $D^4$, $D^6$ and $D^7$, and $D^9$ and $D^{10}$ have chemical structures that are different from each other, and each combination of two $D^1$, three $D^2$, two $D^3$, two $D^4$, three $D^5$, two $D^6$, two $D^8$, and two $D^9$ have chemical structures that are identical with each other. At least one hydrogen atom in the general formulae (1) to (6) and the following general formulae (7) to (13) may be substituted with a deuterium atom.

General Formula (7)

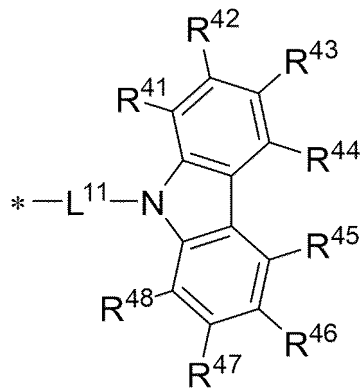

It is preferable that the group represented by general formula (7) is a group represented by any of the following general formulae (8) to (13).

General Formula (8)

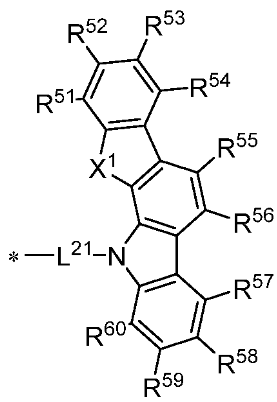

General Formula (9)

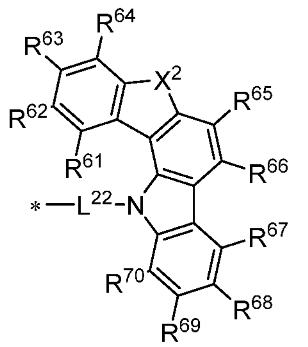

General Formula (10)

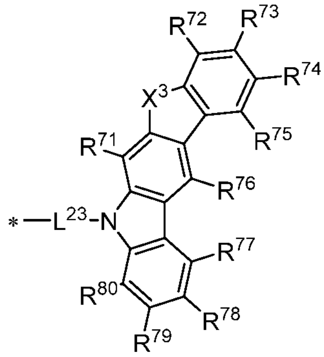

General Formula (11)

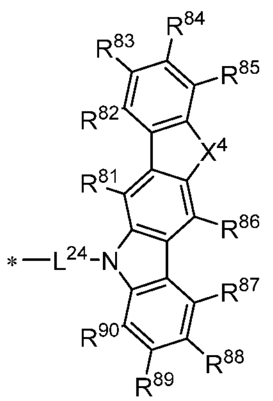

General Formula (12)

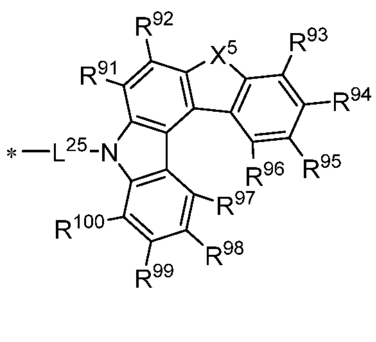

General Formula (13)

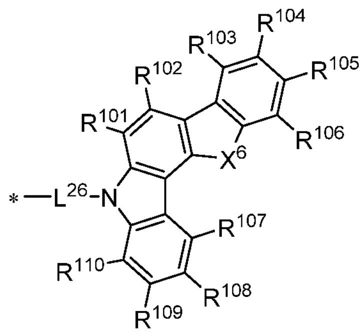

In general formulae (7) to (13), $L^{11}$ and $L^{21}$ to $L^{26}$ each represent a single bond or a divalent linking group. Examples of the divalent linking group include a substituted or unsubstituted arylene group and a substituted or unsubstituted heteroarylene group.

In general formulae (7) to (13), $R^{41}$ to $R^{110}$ each independently represent a hydrogen atom or a substituent. $R^{41}$ and $R^{42}$, $R^{42}$ and $R^{43}$, $R^{43}$ and $R^{44}$, $R^{44}$ and $R^{45}$, $R^{45}$ and $R^{46}$, $R^{46}$ and $R^{47}$, $R^{47}$ and $R^{48}$, $R^{51}$ and $R^{52}$, $R^{52}$ and $R^{53}$, $R^{53}$ and $R^{54}$, $R^{54}$ and $R^{55}$, $R^{55}$ and $R^{56}$, $R^{56}$ and $R^{57}$, $R^{57}$ and $R^{58}$, $R^{58}$ and $R^{59}$, $R^{59}$ and $R^{60}$, $R^{61}$ and $R^{62}$ , $R^{62}$ and $R^{63}$, $R^{63}$ and $R^{64}$, $R^{65}$ and $R^{66}$, $R^{66}$ and $R^{67}$, $R^{67}$ and $R^{68}$, $R^{68}$ and $R^{69}$, $R^{69}$ and $R^{70}$, $R^{72}$ and $R^{73}$, $R^{73}$ and $R^{74}$, $R^{74}$ and $R^{75}$, $R^{75}$ and $R^{76}$, $R^{76}$ and $R^{77}$, $R^{77}$ and $R^{78}$, $R^{78}$ and $R^{79}$, $R^{79}$ and $R^{80}$, $R^{81}$ and $R^{82}$, $R^{82}$ and $R^{83}$, $R^{83}$ and $R^{84}$, $R^{84}$ and $R^{85}$, $R^{86}$ and $R^{87}$, $R^{87}$ and $R^{88}$, $R^{88}$ and $R^{89}$, $R^{89}$ and $R^{90}$, $R^{91}$ and $R^{92}$, $R^{93}$ and $R^{94}$, $R^{94}$ and $R^{95}$, $R^{95}$ and $R^{96}$, $R^{96}$ and $R^{97}$, $R^{97}$ and $R^{98}$, $R^{99}$ and $R^{100}$, $R^{101}$ and $R^{102}$, $R^{102}$ and $R^{103}$, $R^{103}$ and $R^{104}$, $R^{104}$ and $R^{105}$, $R^{105}$ and $R^{106}$, $R^{107}$ and $R^{108}$, $R^{108}$ and $R^{109}$, and $R^{109}$ and $R^{110}$ may be bonded to each other to form a cyclic structure. The cyclic structure formed by bonding to each other may be an aromatic ring or an aliphatic ring and may contain a heteroatom, and the cyclic structure may be a fused ring of two or more rings. The heteroatom as used herein is preferably selected from the group consisting of a nitrogen atom, an oxygen atom, and a sulfur atom. Examples of the cyclic structure to be formed include a benzene ring, a naphthalene ring, a pyridine ring, a pyridazine ring, a pyrimidine ring, a pyrazine ring, a pyrrole ring, an imidazole ring, a pyrazole ring, an imidazoline ring, an oxazole ring, an isoxazole ring, a thiazole ring, an isothiazole, ring, a cyclohexadiene ring, a cyclohexene ring, a cyclopentene ring, a cycloheptatriene ring, a cycloheptadiene ring, a cycloheptene ring, a furan ring, a thiophene ring, a naphthyridine ring, a quinoxaline ring, and a quinoline ring. For example, a ring in which a number of rings are fused, such as a phenanthrene ring or a triphenylene ring, may be formed. The number of rings included in the group represented by general formula (7) may be selected from the range of 3 to 5, or may be selected from the range of 5 to 7. The number of rings included in the group represented by any of general formulae (8) to (13) may be selected from the range of 5 to 7, or may be 5.

Examples of the substituent that may be adopted by $R^{41}$ to $R^{110}$ include groups in the following substituent group B, and the substituent is preferably an unsubstituted alkyl group having 1 to 10 carbon atoms, or an aryl group having 6 to 10 carbon atoms which may be substituted with an unsubstituted alkyl group having 1 to 10 carbon atoms. According to a preferred aspect of the invention, $R^{41}$ to $R^{110}$ are each a hydrogen atom or an unsubstituted alkyl group having 1 to 10 carbon atoms. According to a preferred aspect, $R^{41}$ to $R^{110}$ are each a hydrogen atom or an unsubstituted aryl group having 6 to 10 carbon atoms. According to a preferred aspect of the invention, all of $R^{41}$ to $R^{110}$ are hydrogen atoms.

The carbon atoms to which $R^{41}$ to $R^{110}$ are bonded (ring skeleton-constituting carbon atoms) in general formulae (7) to (13) may be each independently substituted with a nitrogen atom. That is, C-$R^{41}$ to C-$R^{110}$ in general formulae (7) to (13) may be each independently substituted with N. The number of substitutions with a nitrogen atom is preferably 0 to 4, and more preferably 1 or 2, in the group represented by any of general formulae (7) to (13). According to an aspect of the invention, the number of substitutions with a nitrogen atom is 0. Furthermore, when there are two or more are substitutions with a nitrogen atom, the number of substitutions with a nitrogen atom in one ring is preferably 1.

In general formulae (7) to (13), $X^1$ to $X^6$ each represent an oxygen atom, a sulfur atom, or N-R. According to an aspect of the invention, $X^1$ to $X^6$ are each an oxygen atom. According to an aspect of the invention, $X^1$ to $X^6$ are each a sulfur atom. According to an aspect of the invention, $X^1$ to $X^6$ are each N-R. R represents a hydrogen atom or a substituent, and is preferably a substituent. Examples of the substituent include substituents selected from the above-described substituent group A. For example, an unsubstituted phenyl group, or a phenyl group substituted with one group, or a group obtained by combining two or more groups, selected from the group consisting of an alkyl group and an aryl group can be preferably employed.

In general formulae (7) to (13), the symbol * represents a bonding position.

Furthermore, the group represented by general formula (7) may be a substituted or unsubstituted 9-carbazolyl group, or may be an aryl group substituted with a substituted or unsubstituted 9-carbazolyl group, or a heteroaryl group

substituted with a substituted or unsubstituted 9-carbazolyl group. Here, a preferred position of the substituent in the 9-carbazolyl group is at least one of the 3-position and the 6-position.

However, at least one of the groups represented by general formula (7) is preferably a group represented by any of general formulae (8) to (13). That is, it is preferable that at least one of $D^1$ and $D^2$ in general formula (1), at least one of $D^3$ and $D^4$ in general formula (2), $D^5$ in general formula (3), at least one of $D^6$ and $D^7$ in general formula (4), $D^8$ in general formula (5), and at least one of $D^9$ and $D^{10}$ in general formula (6) is a group represented by any of general formulae (8) to (13). Furthermore, in general formulae (1), (2), (4), and (6), it is also preferable that $D^2$, $D^4$, $D^6$, and $D^9$ are each a group represented by any of general formulae (8) to (13), and $D^1$, $D^3$, $D^7$, and $D^{10}$ are each a substituted or unsubstituted 9-carbazolyl group, an aryl group substituted with a substituted or unsubstituted 9-carbazolyl group, or a heteroaryl group substituted with a substituted or unsubstituted 9-carbazolyl group.

In general formulae (2) and (5), $Ar^1$ and $Ar^2$ each independently represent a substituted or unsubstituted aryl group or a substituted or unsubstituted heteroaryl group (provided that a group represented by general formula (7) is excluded). $Ar^1$ and $Ar^2$ are each preferably a substituted or unsubstituted aryl group, more preferably a substituted or unsubstituted phenyl group, and even more preferably an unsubstituted phenyl group or a phenyl group having at least one hydrogen atom substituted with a deuterium atom.

According to a preferred aspect of the invention, the delayed fluorescent material is a compound represented by general formula (2), in which the PBHT(Tn) is PBHT(T3) of the third excited triplet state. According to a more preferred aspect of the invention, the delayed fluorescent material is a compound represented by general formula (2), in which $D^4$ is a group represented by general formula (13), and the PBHT(Tn) is PBHT(T3) of the third excited triplet state.

According to another preferred aspect, the delayed fluorescent material is a compound represented by general formula (4) or (5), in which the PBHT(Tn) is PBHT(T2) of the second excited triplet state. According to another more preferred aspect of the invention, the delayed fluorescent material is a compound represented by general formula (4), in which $D^6$ is a group represented by general formula (13), and the PBHT(Tn) is PBHT(T2) of the second excited triplet state. According to still another more preferred aspect of the invention, the delayed fluorescent material is a compound represented by general formula (5), in which $D^8$ is a group represented by general formula (13), and PBHT(Tn) is PBHT(T2) of the second excited triplet state.

Specific examples of the delayed fluorescent material having a ΔPBHT of 0.01 or more, which can be used in the present invention, will be described below. However, the delayed fluorescent material that can be used in the invention should not be construed as being limited to these specific examples.

Compound T1

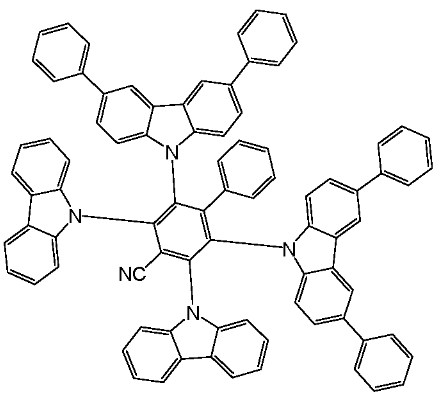

Compound T2

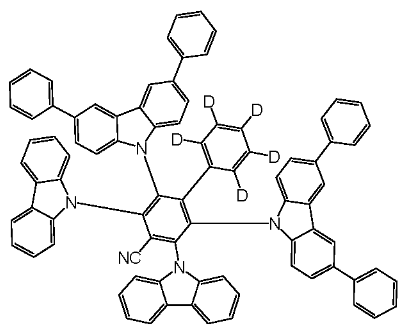

Compound T3

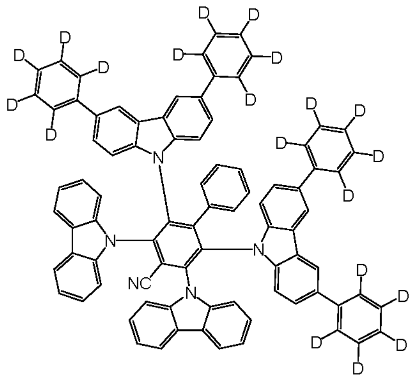

Compound T4

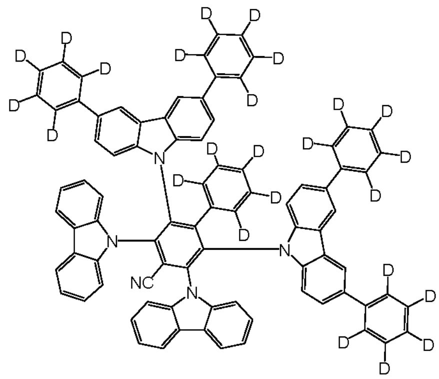

Compound T5

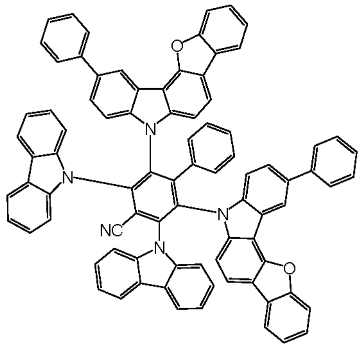

Compound T6

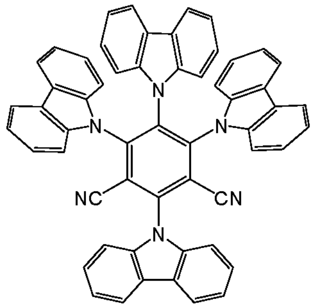

Compound T7

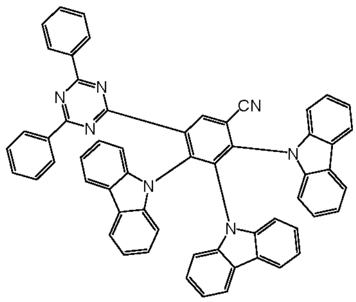

Compound T8

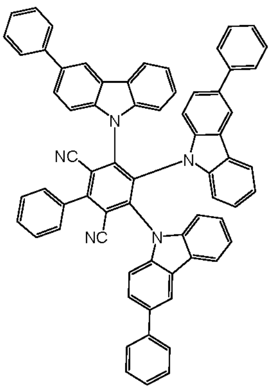

Compound T9

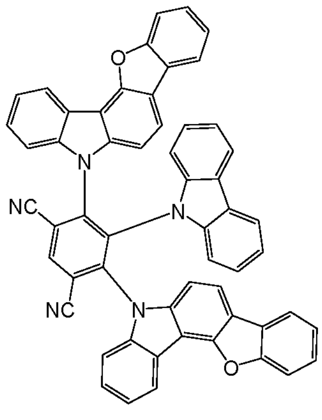

Compound T10

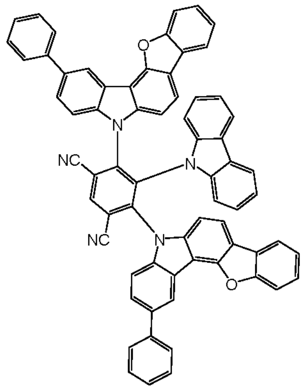

Compound T11

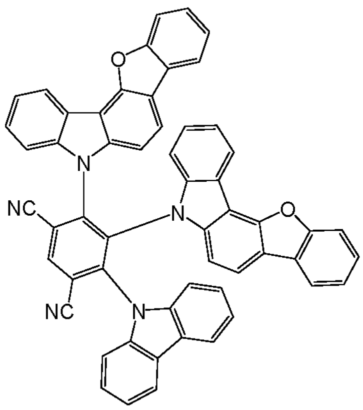

Compound T12

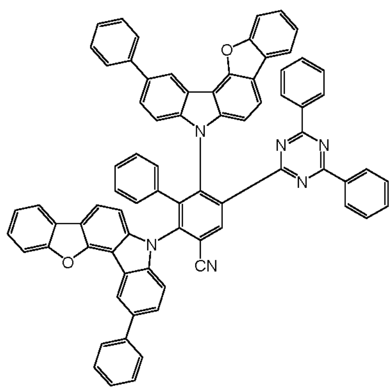

Among the above-described compounds T1 to T12, in the compounds T1 to T5, PBHT(T3) of the third excited triplet state T3 corresponds to the PBHT(Tn), and in the compounds T6 to T12, PBHT(T2) of the second excited triplet state T2 corresponds to the PBHT(Tn).

The "alkyl group" can be any of linear, branched or cyclic ones. Two or more of a linear moiety, a cyclic moiety and a branched moiety can be in the group as mixed. The carbon number of the alkyl group can be, for example, 1 or more, 2 or more, or 4 or more. The carbon number can also be 30 or less, 20 or less, 10 or less, 6 or less, or 4 or less. Specific examples of the alkyl group include a methyl group, an ethyl group, an n-propyl group, an isopropyl group, an n-butyl group, an isobutyl group, a tert-butyl group, an n-pentyl group, an isopentyl group, an n-hexyl group, an isohexyl group, a 2-ethylhexyl group, an n-heptyl group, an isoheptyl group, an n-octyl group, an isooctyl group, an n-nonyl group, an isononyl group, an n-decanyl group, an isodecanyl group, a cyclopentyl group, a cyclohexyl group, and a cycloheptyl group. The alkyl group as a substituent may be further substituted with an aryl group.

The "alkenyl group" can be linear, branched or cyclic. Two or more of a linear moiety, a cyclic moiety and a branched moiety can be in the group as mixed. The carbon number of the alkenyl group can be, for example, 2 or more, or 4 or more. The carbon number can also be 30 or less, 20 or less, 10 or less, 6 or less, or 4 or less. Specific examples of the alkenyl group include an ethenyl group, an n-propenyl group, an isopropenyl group, an n-butenyl group, an isobutenyl group, an n-pentenyl group, an isopentenyl group, an n-hexenyl group, an isohexenyl group, and a 2-ethylhexenyl group. The alkenyl group which is the substituent can be further substituted with a substituent.

The "aryl group" and the "heteroaryl group" may be each a monocyclic ring or may be each a fused ring in which two or more rings are fused. In the case of a fused ring, the number of rings that are fused is preferably 2 to 6, and for example, the number of rings can be selected from 2 to 4. Specific examples of the ring include a benzene ring, a pyridine ring, a pyrimidine ring, a triazine ring, a naphthalene ring, an anthracene ring, a phenanthrene ring, a triphenylene ring, a quinoline ring, a pyrazine ring, a quinoxaline ring, and a naphthyridine ring, and the ring may be a fused ring of these. Specific examples of the aryl group or a heteroaryl group include a phenyl group, a 1-naphthyl group, a 2-naphthyl group, a 1-anthracenyl group, a 2-anthracenyl group, a 9-anthracenyl group, a 2-pyridyl group, a 3-pyridyl group, and a 4-pyridyl group. The number of ring skeleton-constituting atoms of the aryl group is preferably 6 to 40, and more preferably 6 to 20, and may be selected from the range of 6 to 14 or may be selected from the range of 6 to 10. The number of ring skeleton-constituting atoms of the heteroaryl group is preferably 4 to 40, and more preferably 5 to 20, and may be selected from the

range of 5 to 14 or may be selected from the range of 5 to 10. The terms "arylene group" and "heteroarylene group" can be understood to mean groups in which the valence in the description of the aryl group and the heteroaryl group is changed from 1 to 2.

The "substituent group A" in the present specification means one group, or a group obtained by combining two or more groups, selected from the group consisting of a hydroxyl group, a halogen atom (for example, a fluorine atom, a chlorine atom, a bromine atom, or an iodine atom), an alkyl group (for example, having 1 to 40 carbon atoms), an alkoxy group (for example, having 1 to 40 carbon atoms), an alkylthio group (for example, having 1 to 40 carbon atoms), an aryl group (for example, having 6 to 30 carbon atoms), an aryloxy group (for example, having 6 to 30 carbon atoms), an arylthio group (for example, having 6 to 30 carbon atoms), a heteroaryl group (for example, the number of ring skeleton-constituting atoms is 5 to 30), a heteroaryloxy group (for example, the number of ring skeleton-constituting atoms is 5 to 30), a heteroarylthio group (for example, the number of ring skeleton-constituting atoms is 5 to 30), an acyl group (for example, having 1 to 40 carbon atoms), an alkenyl group (for example, having 1 to 40 carbon atoms), an alkynyl group (for example, having 1 to 40 carbon atoms), an alkoxycarbonyl group (for example, having 1 to 40 carbon atoms), an aryloxycarbonyl group (for example, having 1 to 40 carbon atoms), a heteroaryloxycarbonyl group (for example, having 1 to 40 carbon atoms), a silyl group (for example, a trialkylsilyl group having 1 to 40 carbon atoms), and a nitro group.

The "substituent group B" in the present specification means one group, or a group obtained by combining two or more groups, selected from the group consisting of an alkyl group (for example, having 1 to 40 carbon atoms), an alkoxy group (for example, having 1 to 40 carbon atoms), an aryl group (for example having 6 to 30 carbon atoms), an aryloxy group (for example, having 6 to 30 carbon atoms), a heteroaryl group (for example, the number of ring skeleton-constituting atoms is 5 to 30), a heteroaryloxy group (for example, the number of ring skeleton-constituting atoms is 5 to 30), and a diarylaminoamino group (for example, the number of carbon atoms is 0 to 20).

The "substituent group C" in the present specification means one group, or a group obtained by combining two or more groups, selected from the group consisting of an alkyl group (for example, having 1 to 20 carbon atoms), an aryl group (for example, having 6 to 22 carbon atoms), a heteroaryl group (for example, the number of ring skeleton-constituting atoms is 5 to 20), and a diarylamino group (for example, the number of carbon atoms is 12 to 20).

The "substituent group D" in the present specification means one group, or a group obtained by combining two or more groups, selected from the group consisting of an alkyl group (for example, having 1 to 20 carbon atoms), an aryl group (for example, having 6 to 22 carbon atoms), and a heteroaryl group (for example, the number of ring skeleton-constituting atoms is 5 to 20).

The "substituent group E" in the present specification means one group, or a group obtained by combining two or more groups, selected from the group consisting of an alkyl group (for example, having 1 to 20 carbon atoms) and an aryl group (for example, having 6 to 22 carbon atoms).

The substituent described as a "substituent" or a "substituted or unsubstituted" in the present specification may be selected from, for example, the substituent group A, may be selected from the substituent group B, may be selected from the substituent group C, may be selected from the substituent group D, or may be selected from the substituent group E.

In the present specification, the lowest excited singlet energy ($E_{S1}$) and the lowest excited triplet energy ($E_{T1}$) of a compound are values determined by the following procedure. $\Delta E_{ST}$ is a value determined by calculating Esi - $E_{T1}$.

(1) Lowest Excited Singlet Energy ($E_{S1}$)

A thin film or a toluene solution (concentration $10^{-5}$ mol/L) of a compound to be measured is prepared as a sample. The fluorescence spectrum of this sample is measured at normal temperature (300 K). In the fluorescence spectrum, the vertical axis represents light emission, and the horizontal axis represents wavelength. A tangent line is drawn to the rising edge of the short wavelength side of this emission spectrum, and the wavelength value λedge [nm] at the intersection between the tangent line and the horizontal axis is determined. This wavelength value is converted to an energy value using the following conversion formula, and the converted value is designated as Esi.

$$\text{Conversion formula: } E_{S1}\ [eV] = 1239.85/\lambda edge$$

Measurement of the emission spectrum in the Examples that will be described below was carried out using an LED light source (manufactured by Thorlabs, Inc., M300L4) as an excitation light source and using a detector (manufactured by HAMAMATSU PHOTONICS K.K., PMA-12 Multichannel spectrometer C10027-01).

(2) Lowest Excited Triplet Energy ($E_{T1}$)

The same sample as that used for the measurement of the lowest excited singlet energy ($E_{S1}$) is cooled to 77 [K]

using liquid nitrogen, the sample for phosphorescence measurement is irradiated with excitation light (300 nm), and phosphorescence is measured using a detector. The light emission after 100 milliseconds from the irradiation with excitation light is taken as the phosphorescence spectrum. A tangent line is drawn to the rising edge on the short wavelength side of this phosphorescence spectrum, and the wavelength value λedge [nm] at the intersection between the tangent line and the horizontal axis is determined. This wavelength value is converted to an energy value using the following conversion formula, and this converted value is designated as $E_{T1}$.

$$\text{Conversion formula: } E_{T1}\ [eV] = 1239.85/\lambda edge$$

The tangent line to the rising edge on the short wavelength side of the phosphorescence spectrum is drawn as follows. When moving along the spectral curve from the short wavelength side of the phosphorescence spectrum to the maximum value closest to the short wavelength side among the maximum values of the spectrum, a tangent line at each point on the curve toward the long wavelength side is considered. This tangent line increases in slope as the curve rises (that is, as a value of the vertical axis increases). A tangent line drawn at the point where this slope value has the maximum value is defined as a tangent line to the rising edge on the short wavelength side of the phosphorescence spectrum.

Incidentally, a maximum point having a peak intensity of 10% or less of the maximum peak intensity of the spectrum is not included in the above-mentioned maximum value closest to the short wavelength side, and a tangent line drawn at the point that is the closest to the maximum value closest to the short wavelength side, where the slope value is the maximum value, is regarded as a tangent line to the rising edge on the short wavelength side of the phosphorescence spectrum.

[Other Components of Light Emitting Layer]

The light emitting layer may be composed only of a delayed fluorescent material having a ΔPBHT of 0.01 or more, or may contain other components. Examples of the other components include a host material.

(Host Material)

It is preferable that the host material has a function of transporting a carrier. Furthermore, it is preferable that the host material has a function of trapping the energy of the delayed fluorescent material having a ΔPBHT of 0.01 or more within the compound. As a result, the delayed fluorescent material having a ΔPBHT of 0.01 or more can efficiently convert the energy generated by recombination of holes and electrons in the molecule and the energy received from the host material, into light emission. In order to provide the energy trapping function, it is preferable that the host material has a higher lowest excited singlet energy than the delayed fluorescent material having a ΔPBHT of 0.01 or more, and it is more preferable that the host material has both a higher lowest excited singlet energy and a higher lowest excited triplet energy than the delayed fluorescent material having a ΔPBHT of 0.01 or more.

The host material is preferably an organic compound that has a hole transporting ability and an electron transporting ability, prevents the emission wavelength from becoming longer, and has a high glass transition temperature. Furthermore, according to a preferred aspect of the invention, the host material is selected from compounds that do not emanate delayed fluorescence. The light emission from the host material is preferably less than 1%, and more preferably less than 0.1%, of the light emission from the organic electroluminescent device of the invention, and the light emission may be, for example, less than 0.01%, or below the detection limit.

It is preferable that the host material does not contain a metal atom. For example, a compound consisting of atoms selected from the group consisting of a carbon atom, a hydrogen atom, a nitrogen atom, an oxygen atom, and a sulfur atom can be selected as the host material. For example, a compound consisting of atoms selected from the group consisting of a carbon atom, a hydrogen atom, a nitrogen atom, and an oxygen atom can be selected as the host material. For example, a compound consisting of a carbon atom, a hydrogen atom, and a nitrogen atom can be selected as the host material.

Preferred compounds that can be used as the host material will be listed below.

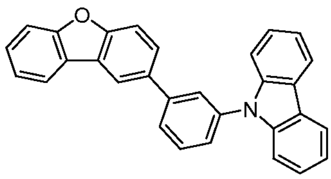

H1

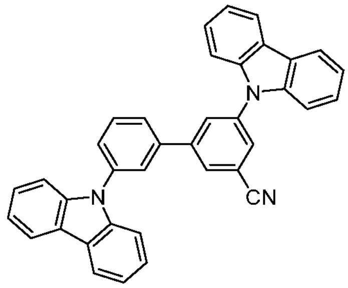

H2

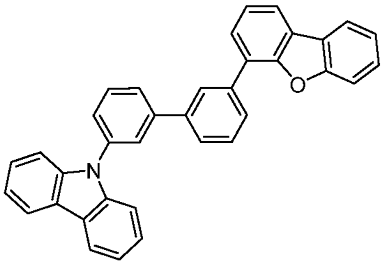

H3

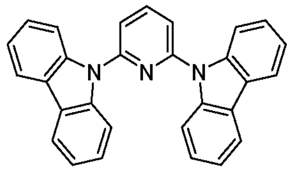

H4

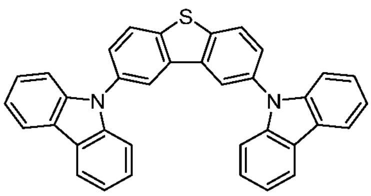

H5

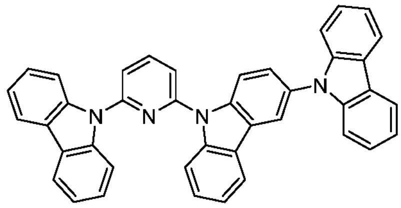

H6

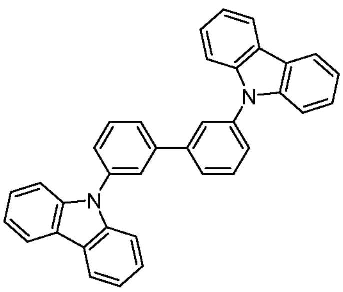

H7

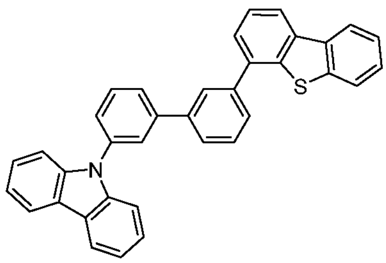

H8

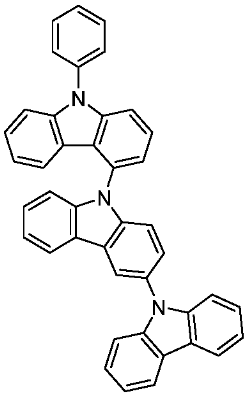

H9

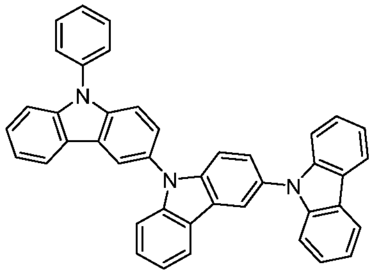

H10

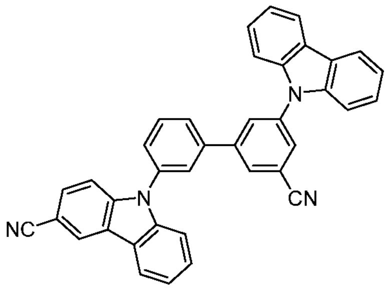

H11

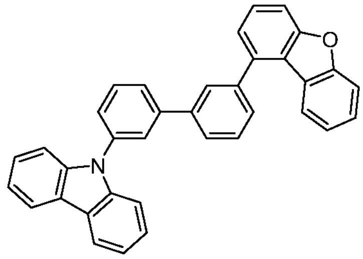

H12

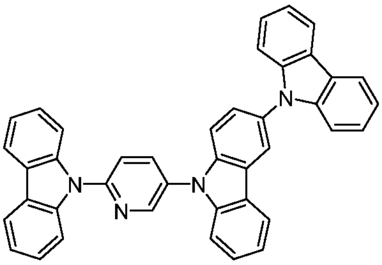

H13

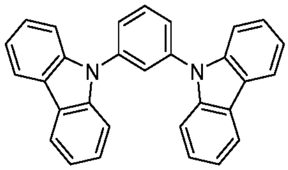

H14

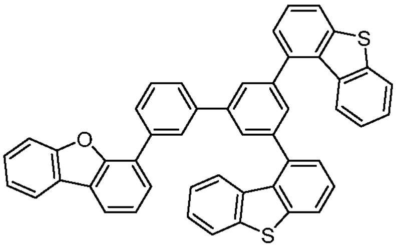

H15

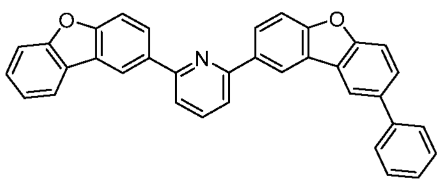

H16

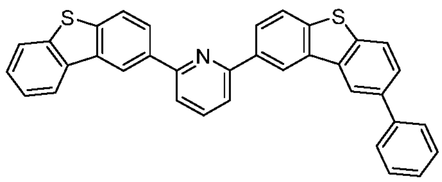

H17

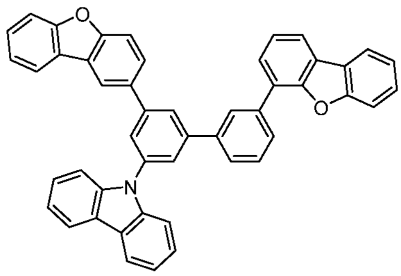

H18

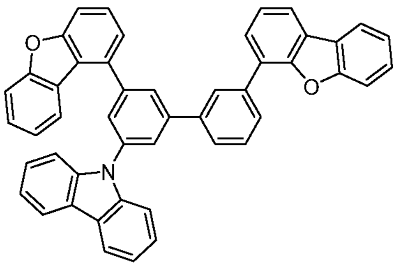

H19

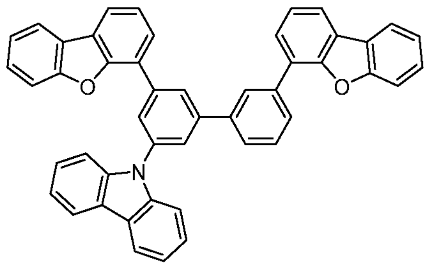

H20

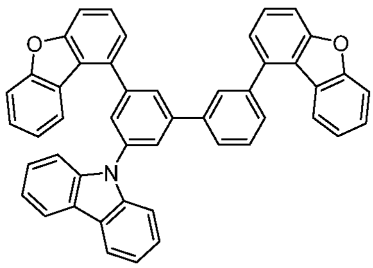

H21

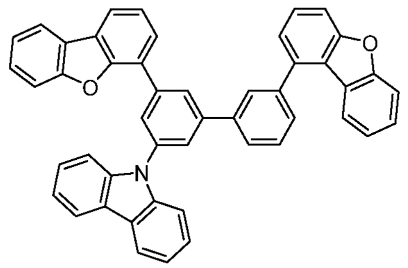

H22

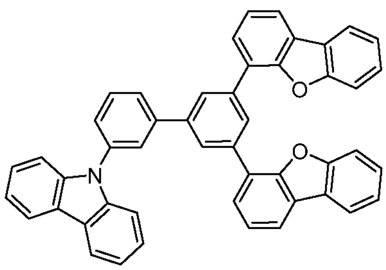

H23

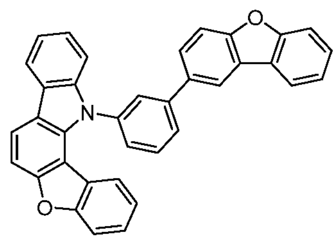

H24

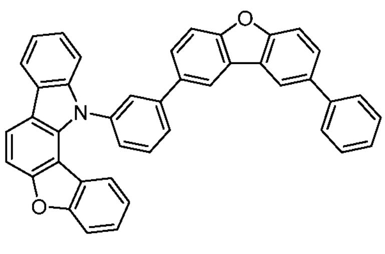

H25

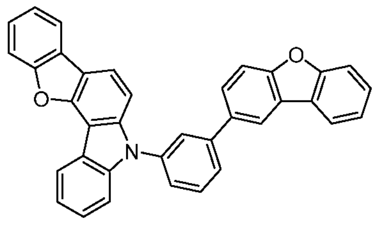

H26

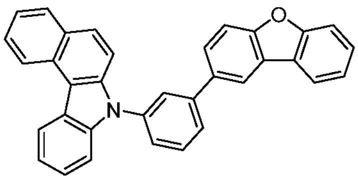

H27

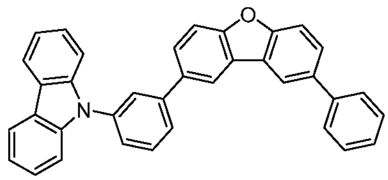

H28

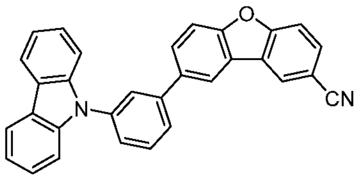

H29

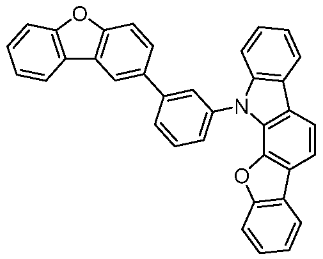

H30

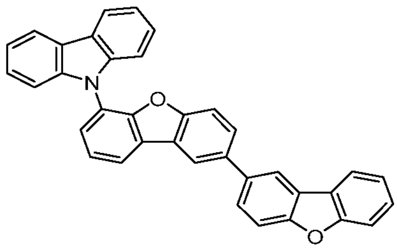

H31

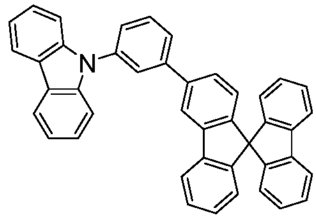

H32

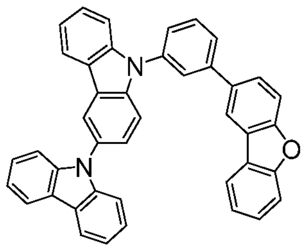

H33

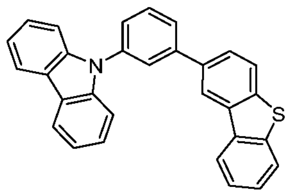

H34

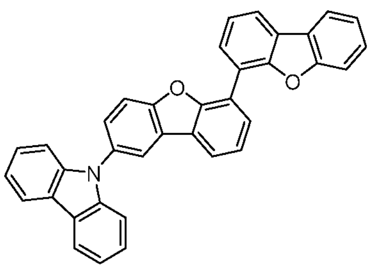

H35

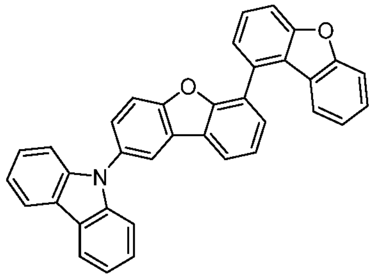

H36

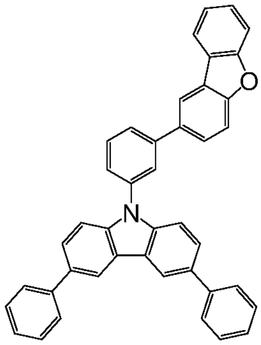

H37

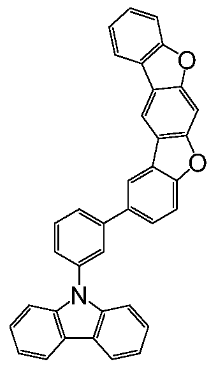

H38

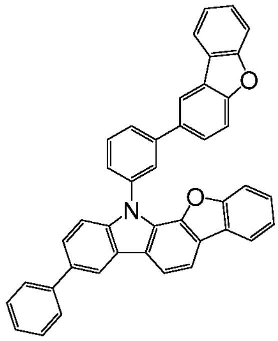

H39

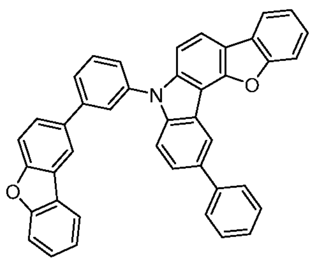

H40

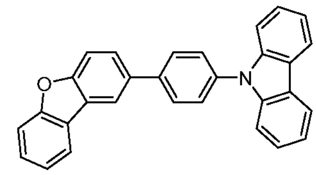

H41

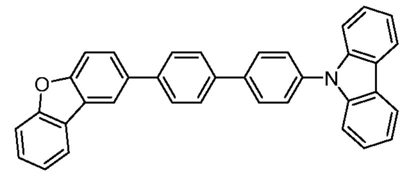

H42

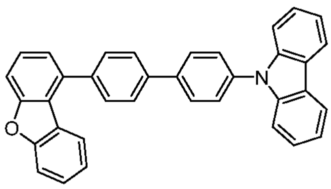

H43

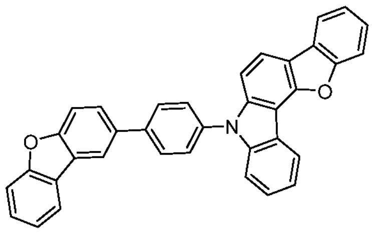

H44

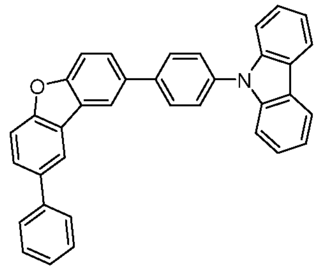

H45

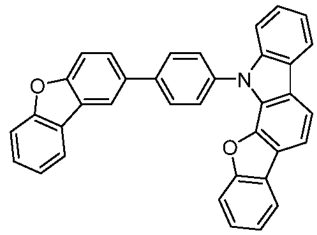

H46

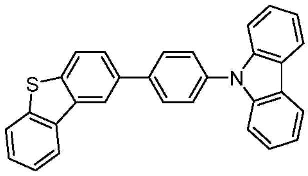

H47

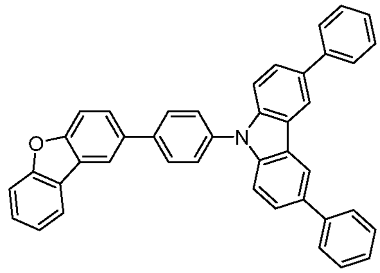

H48

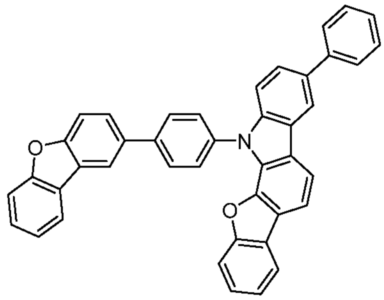

H49

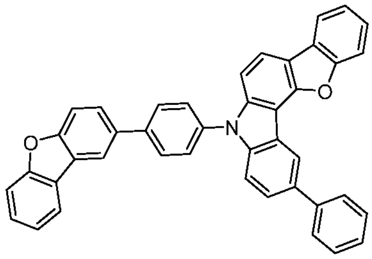

H50

When the host material is used, the amount of the delayed fluorescent material having a ΔPBHT of 0.01 or more contained in the light emitting layer is preferably 0.1% by weight or more, and more preferably 1% by weight or more, and is preferably 50% by weight or less, more preferably 20% by weight or less, and even more preferably 10% by weight or less.

[Overall Configuration of Organic Light Emitting Device]

The organic light emitting device of the invention may be an organic photoluminescent device or an organic electroluminescent device. An organic photoluminescent device (organic PL device) has a structure in which at least a light emitting layer is formed on a substrate. An organic electroluminescent device has an anode, a cathode, and at least one organic layer including a light emitting layer between the anode and the cathode. The organic layer constituting the organic electroluminescent device may be composed only of a light emitting layer, or may include an organic layer other than the light emitting layer. For example, an organic layer may or may not be interposed between the anode and the light emitting layer and between the light emitting layer and the cathode. In other words, the anode and the light emitting layer may be laminated so as to be in direct contact with each other, or may be laminated so as not to be in direct contact with each other. Furthermore, the light emitting layer and the cathode may be laminated so as to be in direct contact with each other, or may be laminated so as not to be in direct contact with each other. The light emitting layer is located between the anode and the cathode, and it is preferable that the light emitting layer is disposed so that the entire light emitting layer is not protruding into the area between the anode and the cathode.

The organic electroluminescent device of the invention may have a substrate that supports an anode, a cathode, and at least one organic layer including a light emitting layer. In this case, the substrate may be disposed on the anode on the opposite side of the light emitting layer, or may be disposed on the cathode on the opposite side of the light emitting

layer. Furthermore, the organic electroluminescent device of the invention may be a top emission type device in which most of the light is emitted through the side opposite from the substrate, or may be a bottom emission type device in which most of the light is emitted through the substrate side. Here, the phrase "most of the light" means 60% or more of the amount of light emitted from the device.

The constituent members and layers of the organic electroluminescent device will be described below. The description of the substrate and the light emitting layer also applies to the substrate and the light emitting layer of the organic photoluminescent device.

(Light Emitting Layer)

The light emitting layer of the organic light emitting device of the invention contains a delayed fluorescent material having a ΔPBHT of 0.01 or more. The light emitting layer may further contain a host material. For the description of the delayed fluorescent material having a ΔPBHT of 0.01 or more, reference can be made to the description in the above-described section [Delayed Fluorescent Material], and for the description of the host material, reference can be made to the description in the above-described section (Host Material). The light emitting layer can be configured not to contain any compound that transfers electric charge or energy or any metal element other than boron, in addition to the delayed fluorescent material having a ΔPBHT of 0.01 or more and the host material. Furthermore, the light emitting layer may be composed only of a compound consisting of atoms selected from the group consisting of a carbon atom, a hydrogen atom, a deuterium atom, a nitrogen atom, a boron atom, an oxygen atom, and a sulfur atom. For example, the light emitting layer can be composed only of a compound consisting of atoms selected from the group consisting of a carbon atom, a hydrogen atom, a deuterium atom, a nitrogen atom, a boron atom, and an oxygen atom. For example, the light emitting layer can be composed only of a compound consisting of atoms selected from the group consisting of a carbon atom, a hydrogen atom, a deuterium atom, a nitrogen atom, a boron atom, and a sulfur atom. For example, the light emitting layer can be composed only of a compound consisting of atoms selected from the group consisting of a carbon atom, a hydrogen atom, a deuterium atom, a nitrogen atom, and a boron atom. For example, the light emitting layer can be composed only of a compound consisting of atoms selected from the group consisting of a carbon atom, a hydrogen atom, a deuterium atom, a nitrogen atom, an oxygen atom, and a sulfur atom. For example, the light emitting layer can be composed only of a compound consisting of atoms selected from the group consisting of a carbon atom, a hydrogen atom, a deuterium atom, and a nitrogen atom. The light emitting layer may contain a delayed fluorescent material composed of atoms selected from the group consisting of a carbon atom, a hydrogen atom, a deuterium atom, a nitrogen atom, an oxygen atom, and a sulfur atom. Furthermore, the light emitting layer may contain a delayed fluorescent material composed of atoms selected from the group consisting of a carbon atom, a hydrogen atom, a deuterium atom, and a nitrogen atom. The light emitting layer may contain a host material composed of atoms selected from the group consisting of a carbon atom, a hydrogen atom, a nitrogen atom, and an oxygen atom, and a delayed fluorescent material composed of atoms selected from the group consisting of a carbon atom, a hydrogen atom, a deuterium atom, a nitrogen atom, an oxygen atom, and a sulfur atom. Furthermore, the light emitting layer may contain a host material composed of atoms selected from the group consisting of a carbon atom, a hydrogen atom, a nitrogen atom, and an oxygen atom, and a delayed fluorescent material composed of atoms selected from the group consisting of a carbon atom, a hydrogen atom, a deuterium atom, and a nitrogen atom.

The light emitting layer may be formed by vapor depositing a delayed fluorescent material having a ΔPBHT of 0.01 or more, may be formed by co-depositing a delayed fluorescent material having a ΔPBHT of 0.01 or more and a host material, may be formed by mixing in advance a delayed fluorescent material having a ΔPBHT of 0.01 or more and a host material to prepare a mixture and vapor depositing the mixture as a deposition source, or may be formed using a solution obtained by dissolving a delayed fluorescent material having a ΔPBHT of 0.01 or more, or a solution obtained by dissolving a delayed fluorescent material having a ΔPBHT of 0.01 or more and a host material, by a coating method.

In the following, the constituent members and the other layers than the light emitting layer of the organic electroluminescent device are described.

Substrate:

In some embodiments, the organic electroluminescent device of the invention is supported by a substrate, wherein the substrate is not particularly limited and can be any of those that have been commonly used in an organic electroluminescent device, for example those formed of glass, transparent plastics, quartz and silicon.

Anode:

In some embodiments, the anode of the organic electroluminescent device is made of a metal, an alloy, an electroconductive compound, or a combination thereof. In some embodiments, the metal, alloy, or electroconductive compound has a large work function (4 eV or more). In some embodiments, the metal is Au. In some embodiments, the

electroconductive transparent material is selected from CuI, indium tin oxide (ITO), $SnO_2$, and ZnO. In some embodiments, an amorphous material capable of forming a transparent electroconductive film, such as IDIXO ($In_2O_3$-ZnO), is be used. In some embodiments, the anode is a thin film. In some embodiments, the thin film is made by vapor deposition or sputtering. In some embodiments, the film is patterned by a photolithography method. In some embodiments, where the pattern may not require high accuracy (for example, approximately 100 μm or more), the pattern can be formed with a mask having a desired shape on vapor deposition or sputtering of the electrode material. In some embodiments, when a material can be applied as a coating, such as an organic electroconductive compound, a wet film forming method, such as a printing method or a coating method is used. In some embodiments, when the emitted light goes through the anode, the anode has a transmittance of more than 10%, and the anode has a sheet resistance of several hundred Ohm per unit area or less. In some embodiments, the thickness of the anode is from 10 to 1000 nm. In some embodiments, the thickness of the anode is from 10 to 200 nm. In some embodiments, the thickness of the anode varies depending on the material used.

Cathode:

In some embodiments, the cathode is made of an electrode material such as a metal having a small work function (4 eV or less) (referred to as an electron injection metal), an alloy, an electroconductive compound, or a combination thereof. In some embodiments, the electrode material is selected from sodium, a sodium-potassium alloy, magnesium, lithium, a magnesium-copper mixture, a magnesium-silver mixture, a magnesium-aluminum mixture, a magnesium-indium mixture, an aluminum-aluminum oxide ($Al_2O_3$) mixture, indium, a lithium-aluminum mixture, and a rare earth element. In some embodiments, a mixture of an electron injection metal and a second metal that is a stable metal having a larger work function than the electron injection metal is used. In some embodiments, the mixture is selected from a magnesium-silver mixture, a magnesium-aluminum mixture, a magnesium-indium mixture, an aluminum-aluminum oxide ($Al_2O_3$) mixture, a lithium-aluminum mixture, and aluminum. In some embodiments, the mixture increases the electron injection property and the durability against oxidation. In some embodiments, the cathode is produced by forming the electrode material into a thin film by vapor deposition or sputtering. In some embodiments, the cathode has a sheet resistance of several hundred Ohm per unit area or less. In some embodiments, the thickness of the cathode ranges from 10 nm to 5 μm. In some embodiments, the thickness of the cathode ranges from 50 to 200 nm. In some embodiments, for transmitting the emitted light, any one of the anode and the cathode of the organic electroluminescent device is transparent or translucent. In some embodiments, the transparent or translucent electroluminescent devices enhances the light emission luminance.

In some embodiments, the cathode is formed with an electroconductive transparent material, as described for the anode, to form a transparent or translucent cathode. In some embodiments, a device comprises an anode and a cathode, both being transparent or translucent.

Injection Layer:

An injection layer is a layer between the electrode and the organic layer. In some embodiments, the injection layer decreases the drive voltage and enhances the light emission luminance. In some embodiments, the injection layer includes a hole injection layer and an electron injection layer. The injection layer can be positioned between the anode and the light emitting layer or the hole transporting layer, and between the cathode and the light emitting layer or the electron transporting layer. In some embodiments, an injection layer is present. In some embodiments, no injection layer is present.

Preferred compound examples for use as a hole injection material are shown below.

MoOs,

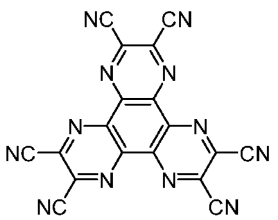
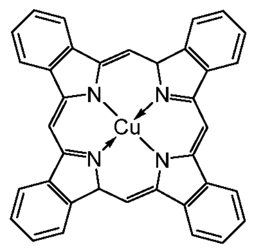
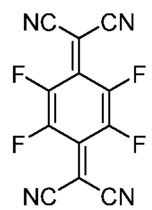
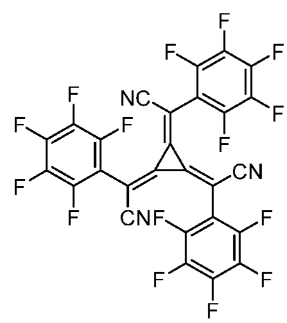

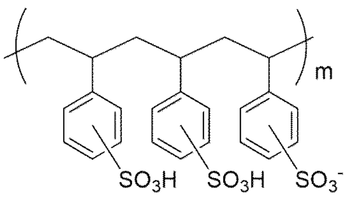 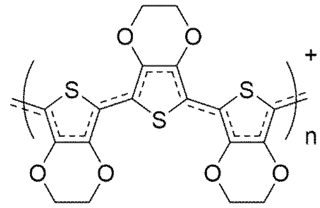

Next, preferred compound examples for use as an electron injection material are shown below.
LiF, CsF,

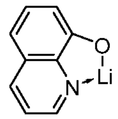

Barrier Layer:

A barrier layer is a layer capable of inhibiting charges (electrons or holes) and/or excitons present in the light emitting layer from being diffused outside the light emitting layer. In some embodiments, the electron barrier layer is between the light emitting layer and the hole transporting layer, and inhibits electrons from passing through the light emitting layer toward the hole transporting layer. In some embodiments, the hole barrier layer is between the light emitting layer and the electron transporting layer, and inhibits holes from passing through the light emitting layer toward the electron transporting layer. In some embodiments, the barrier layer inhibits excitons from being diffused outside the light emitting layer. In some embodiments, the electron barrier layer and the hole barrier layer are exciton barrier layers. As used herein, the term "electron barrier layer" or "exciton barrier layer" includes a layer that has both the function of an electron barrier layer and the function of an exciton barrier layer.

Hole Barrier Layer:

A hole barrier layer acts as an electron transporting layer. In some embodiments, the hole barrier layer inhibits holes from reaching the electron transporting layer while transporting electrons. In some embodiments, the hole barrier layer enhances the recombination probability of electrons and holes in the light emitting layer. The material used for the hole barrier layer can be the same materials as the ones described for the electron transporting layer.
Preferred compound examples for use for the hole barrier layer are shown below.

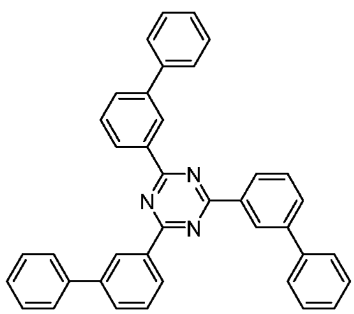 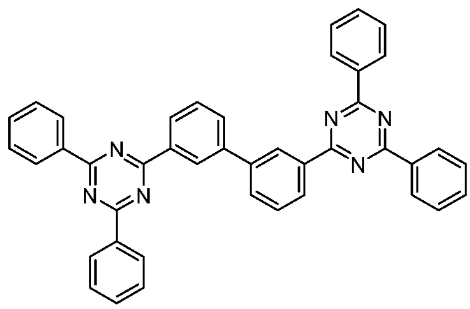

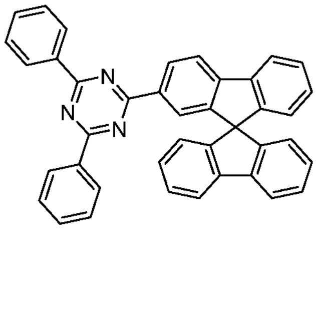 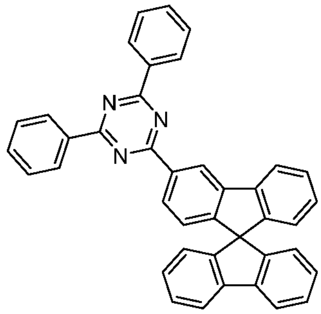

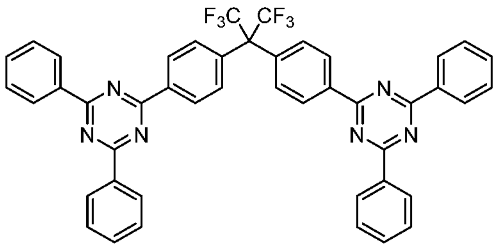

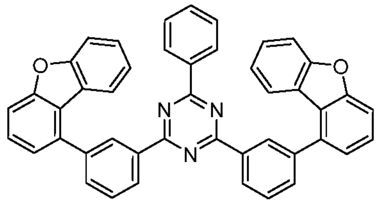

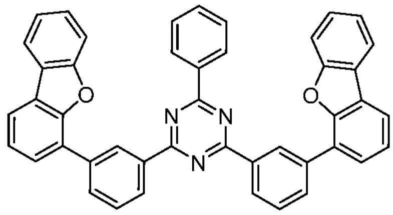

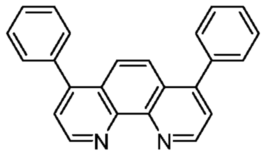

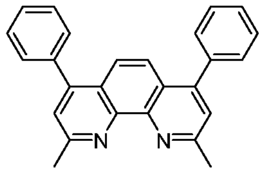

Electron Barrier Layer:

An electron barrier layer transports holes. In some embodiments, the electron barrier layer inhibits electrons from reaching the hole transporting layer while transporting holes. In some embodiments, the electron barrier layer enhances the recombination probability of electrons and holes in the light emitting layer. The material used for the electron barrier layer can be the same material as the ones described above for the hole transporting layer.

Preferred compound examples for use as the electron barrier material are shown below.

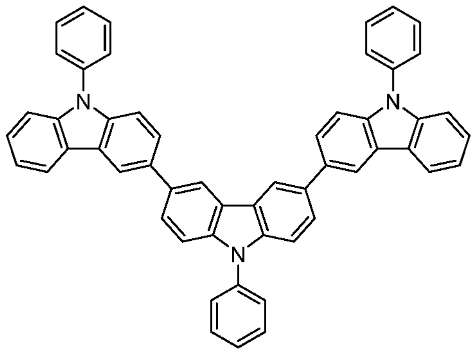

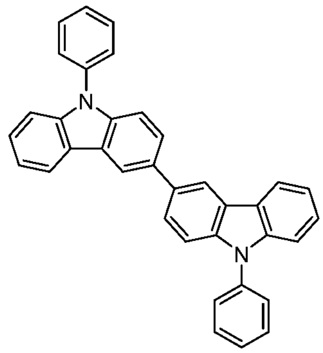

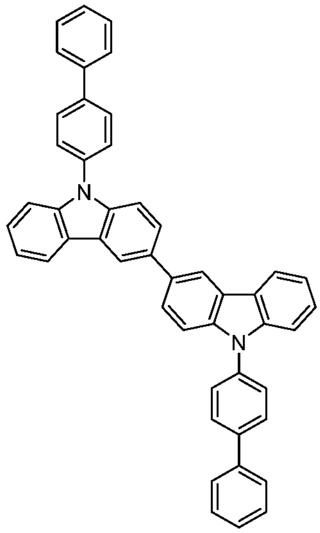

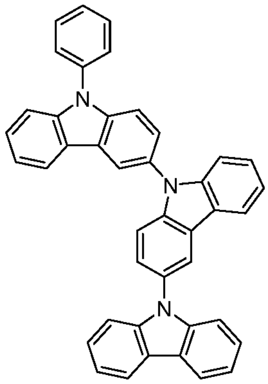

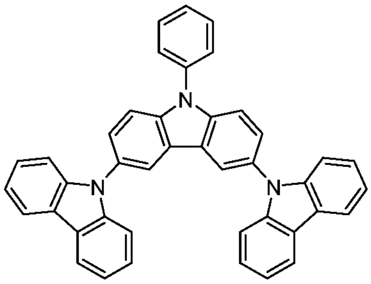

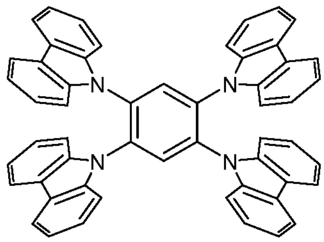

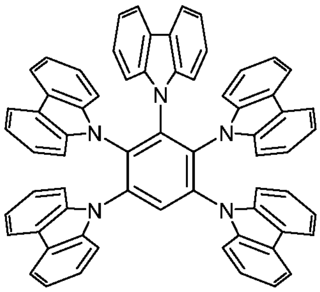

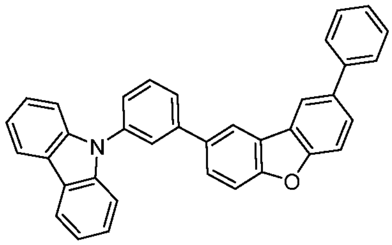

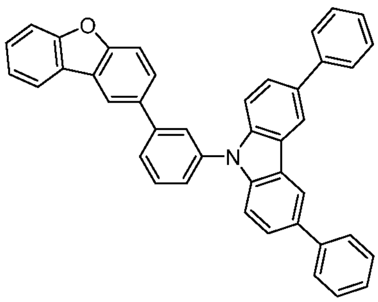

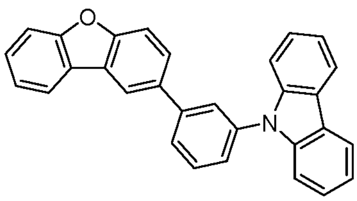

Exciton Barrier Layer:

An exciton barrier layer inhibits excitons generated through recombination of holes and electrons in the light emitting layer from being diffused to the charge transporting layer. In some embodiments, the exciton barrier layer enables effective confinement of excitons in the light emitting layer. In some embodiments, the light emission efficiency of the device is enhanced. In some embodiments, the exciton barrier layer is adjacent to the light emitting layer on any of the side of the anode and the side of the cathode, and on both the sides. In some embodiments, where the exciton barrier layer is on the side of the anode, the layer can be between the hole transporting layer and the light emitting layer and adjacent to the

light emitting layer. In some embodiments, where the exciton barrier layer is on the side of the cathode, the layer can be between the light emitting layer and the cathode and adjacent to the light emitting layer. In some embodiments, a hole injection layer, an electron barrier layer, or a similar layer is between the anode and the exciton barrier layer that is adjacent to the light emitting layer on the side of the anode. In some embodiments, a hole injection layer, an electron barrier layer, a hole barrier layer, or a similar layer is between the cathode and the exciton barrier layer that is adjacent to the light emitting layer on the side of the cathode. In some embodiments, the exciton barrier layer comprises excited singlet energy and excited triplet energy, at least one of which is higher than the excited singlet energy and the excited triplet energy of the light emitting material, respectively.

Hole Transporting Layer:

The hole transporting layer comprises a hole transporting material. In some embodiments, the hole transporting layer is a single layer. In some embodiments, the hole transporting layer comprises a plurality of layers.

In some embodiments, the hole transporting material has one of injection or transporting property of holes and barrier property of electrons. In some embodiments, the hole transporting material is an organic material. In some embodiments, the hole transporting material is an inorganic material. Examples of known hole transporting materials that can be used in the present invention include but are not limited to a triazole derivative, an oxadiazole derivative, an imidazole derivative, a carbazole derivative, an indolocarbazole derivative, a polyarylalkane derivative, a pyrazoline derivative, a pyrazolone derivative, a phenylenediamine derivative, an allylamine derivative, an amino-substituted chalcone derivative, an oxazole derivative, a styrylanthracene derivative, a fluorenone derivative, a hydrazone derivative, a stilbene derivative, a silazane derivative, an aniline copolymer and an electroconductive polymer oligomer (particularly, a thiophene oligomer), or a combination thereof. In some embodiments, the hole transporting material is selected from a porphyrin compound, an aromatic tertiary amine compound, and a styrylamine compound. In some embodiments, the hole transporting material is an aromatic tertiary amine compound. Preferred compound examples for use as the hole transporting material are shown below.

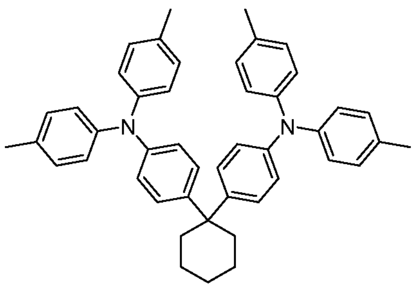

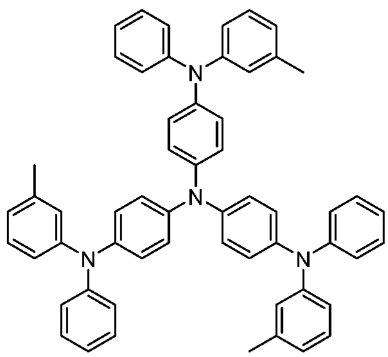

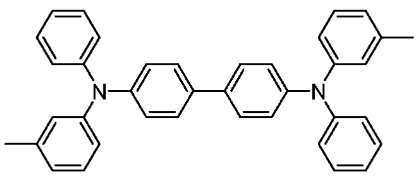

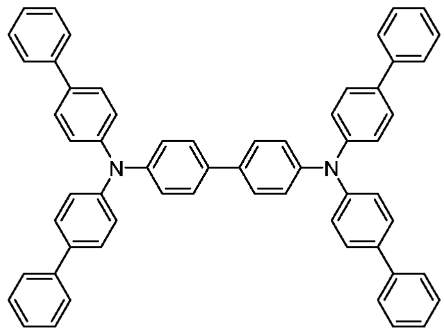

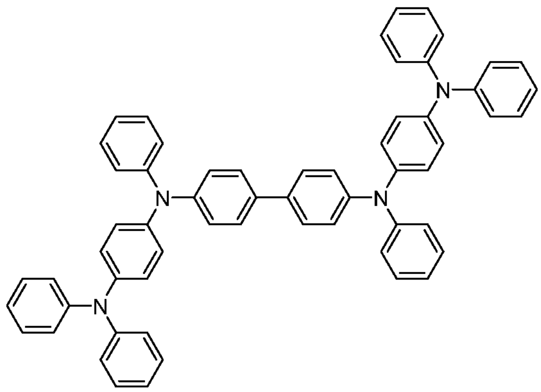

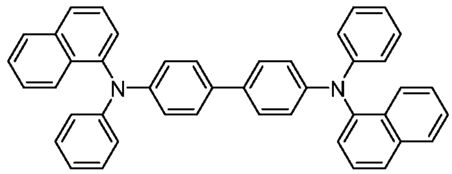

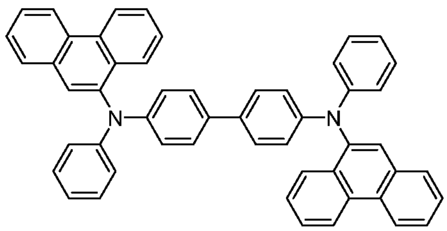

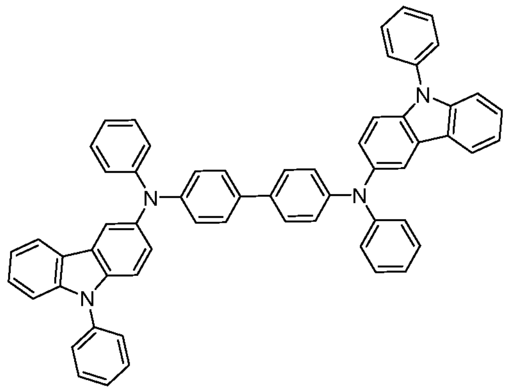

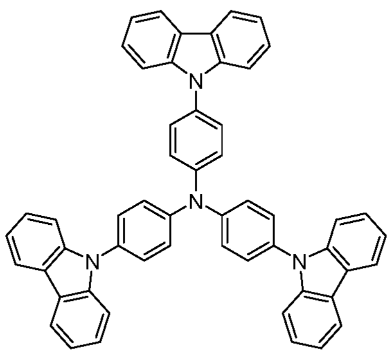

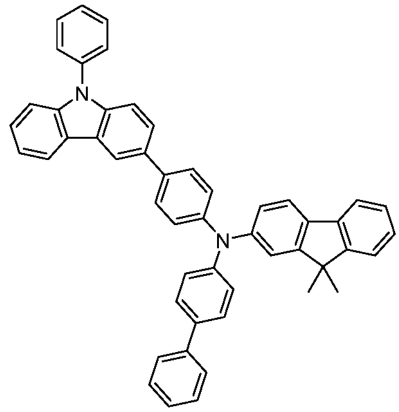

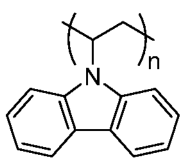

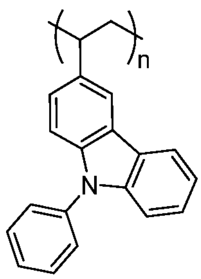

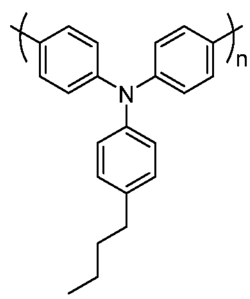

Electron Transporting Layer:

The electron transporting layer comprises an electron transporting material. In some embodiments, the electron transporting layer is a single layer. In some embodiments, the electron transporting layer comprises a plurality of layers.

In some embodiments, the electron transporting material needs only to have a function of transporting electrons,

which are injected from the cathode, to the light emitting layer. In some embodiments, the electron transporting material also function as a hole barrier material. Examples of the electron transporting layer that can be used in the present invention include but are not limited to a nitro-substituted fluorene derivative, a diphenylquinone derivative, a thiopyran dioxide derivative, carbodiimide, a fluorenylidene methane derivative, anthraquinodimethane, an anthrone derivatives, an oxadiazole derivative, an azole derivative, an azine derivative, or a combination thereof, or a polymer thereof. In some embodiments, the electron transporting material is a thiadiazole derivative, or a quinoxaline derivative. In some embodiments, the electron transporting material is a polymer material. Preferred compound examples for use as the electron transporting material are shown below.

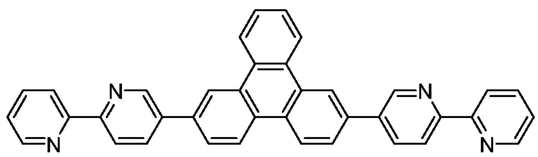

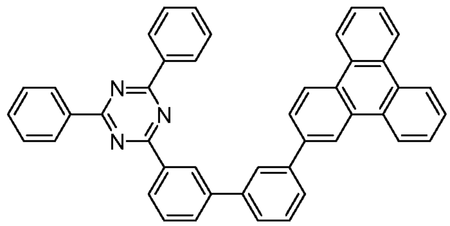

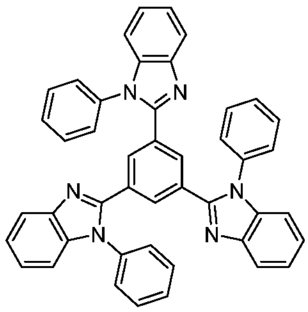

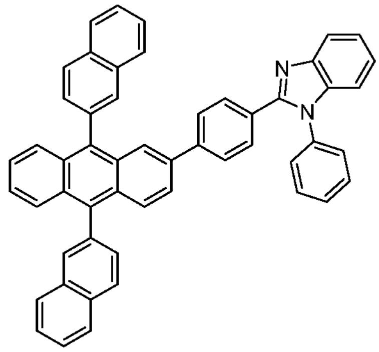

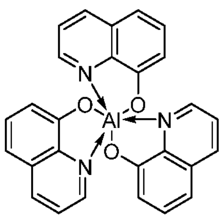

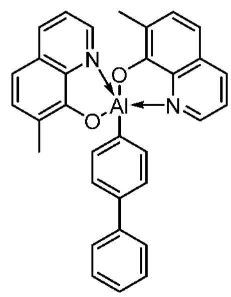

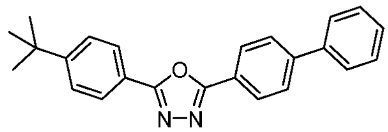

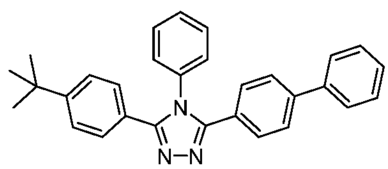

Hereinunder, compound examples preferred as a material that can be added to the organic layers are shown. For example, it is conceivable to add these as a stabilization material.

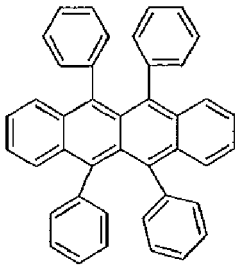 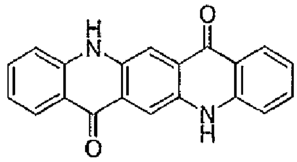 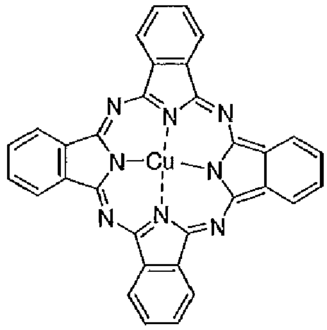

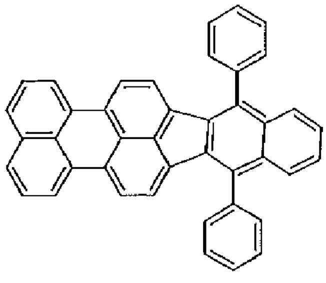 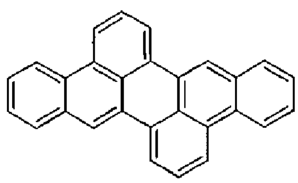

Preferred materials for use in the organic electroluminescent device are specifically shown. However, the materials usable in the invention should not be limitatively interpreted by the following exemplary compounds. Compounds that are exemplified as materials having a specific function can also be used as materials having any other function.

Device:

In some embodiments, a light emitting layer is incorporated into a device. For example, the device includes, but is not limited to an OLED bulb, an OLED lamp, a television screen, a computer monitor, a mobile phone, and a tablet.

In some embodiments, an electronic device includes an OLED comprising an anode, a cathode, and at least one organic layer comprising a light emitting layer between the anode and the cathode.

In some embodiments, compositions described herein can be incorporated into various light-sensitive or light-activated devices, such as OLEDs or opto-electronic devices. In some embodiments, the composition can be useful in facilitating charge transfer or energy transfer within a device and/or as a hole-transport material. The device can be, for example, an organic light emitting diode (OLED), an organic integrated circuit (O-IC), an organic field-effect transistor (O-FET), an organic thin-film transistor (O-TFT), an organic light emitting transistor (O-LET), an organic solar cell (O-SC), an organic optical detector, an organic photoreceptor, an organic field-quench device (O-FQD), a light emitting electrochemical cell (LEC) or an organic laser diode (O-laser).

Bulb or Lamp:

In some embodiments, an electronic device includes an OLED comprising an anode, a cathode, and at least one organic layer comprising a light emitting layer between the anode and the cathode.

In some embodiments, a device comprises OLEDs that differ in color. In some embodiments, a device comprises an array comprising a combination of OLEDs. In some embodiments, the combination of OLEDs is a combination of three colors (e.g., RGB). In some embodiments, the combination of OLEDs is a combination of colors that are not red, green, or blue (for example, orange and yellow green). In some embodiments, the combination of OLEDs is a combination of two, four, or more colors.

In some embodiments, a device is an OLED light comprising:

a circuit board having a first surface with a mounting surface and an opposing second surface, and defining at least one opening;

at least one OLED on the mounting surface, the at least one OLED configured to emanate light, the OLED comprising: an anode, a cathode, and at least one organic layer comprising a light emitting layer between the anode and the cathode;

a housing for the circuit board; and

at least one connector arranged at an end of the housing, the housing and the connector defining a package adapted

for installation in a light fixture.

In some embodiments, the OLED light comprises a plurality of OLEDs mounted on a circuit board such that light emanates in a plurality of directions. In some embodiments, a portion of the light emanated in a first direction is deflected to emanate in a second direction. In some embodiments, a reflector is used to deflect the light emanated in a first direction.

Display or Screen:

In some embodiments, the light emitting layer of the invention can be used in a screen or a display. In some embodiments, the compounds of the invention are deposited onto a substrate using a process including, but not limited to, vacuum evaporation, deposition, vapor deposition, or chemical vapor deposition (CVD). In some embodiments, the substrate is a photoplate structure useful in a two-sided etching that provides a unique aspect ratio pixel. The screen (which can also be referred to as a mask) is used in a process in the manufacturing of OLED displays. The corresponding artwork pattern design facilitates a very steep and narrow tie-bar between the pixels in the vertical direction and a large, sweeping bevel opening in the horizontal direction. This allows the fine patterning of pixels needed for high resolution displays while optimizing the chemical deposition onto a TFT backplane.

The internal patterning of the pixel allows the construction of a three-dimensional pixel opening with varying aspect ratios in the horizontal and vertical directions. Additionally, the use of imaged "stripes" or halftone circles within the pixel area inhibits etching in specific areas until these specific patterns are undercut and fall off the substrate. At that point the entire pixel area is subjected to a similar etching rate but the depths are varying depending on the halftone pattern. Varying the size and spacing of the halftone pattern allow etching to be inhibited at different rates within the pixel and allow a localized deeper etch needed to create steep vertical bevels.

A preferred material for the deposition mask is invar. Invar is a metal alloy that is cold rolled into a long thin sheet in a steel mill. Invar cannot be electrodeposited onto a rotating mandrel as the nickel mask. A preferred and more cost feasible method for forming the opening areas in the mask used for deposition is through a wet chemical etching.

In some embodiments, a screen or display pattern is a pixel matrix on a substrate. In some embodiments, a screen or display pattern is fabricated using lithography (e.g., photolithography and e-beam lithography). In some embodiments, a screen or display pattern is fabricated using a wet chemical etching. In further embodiments, a screen or display pattern is fabricated using plasma etching.

Method for Manufacturing Device:

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light emitting layer, a counter electrode, and an encapsulation layer, and then is cut from the mother panel.

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light emitting layer, a counter electrode, and an encapsulation layer, and then is cut from the mother panel.

In another aspect, provided herein is a method of manufacturing an organic light emitting diode (OLED) display, the method comprising:

forming a barrier layer on a base substrate of a mother panel;
forming a plurality of display units in units of cell panels on the barrier layer;
forming an encapsulation layer on each of the display units of the cell panels;
applying an organic film to an interface portion between the cell panels.

In some embodiments, the barrier layer is an inorganic film formed of, for example, SiNx, and an edge portion of the barrier layer is covered with an organic film formed of polyimide or acryl. In some embodiments, the organic film helps the mother panel to be softly cut in units of the cell panel.

In some embodiments, the thin film transistor (TFT) layer includes a light emitting layer, a gate electrode, and a source/drain electrode. Each of the plurality of display units may include a thin film transistor (TFT) layer, a planarization film formed on the TFT layer, and a light emitting unit formed on the planarization film, wherein the organic film applied to the interface portion is formed of a same material as a material of the planarization film and is formed at a same time as the planarization film is formed. In some embodiments, a light emitting unit is connected to the TFT layer with a passivation

layer and a planarization film therebetween and an encapsulation layer that covers and protects the light emitting unit. In some embodiments of the method of manufacturing, the organic film contacts neither the display units nor the encapsulation layer.

Each of the organic film and the planarization film can include any one of polyimide and acryl. In some embodiments, the barrier layer can be an inorganic film. In some embodiments, the base substrate can be formed of polyimide. The method can further include, before the forming of the barrier layer on one surface of the base substrate formed of polyimide, attaching a carrier substrate formed of a glass material to another surface of the base substrate, and before the cutting along the interface portion, separating the carrier substrate from the base substrate. In some embodiments, the OLED display is a flexible display.

In some embodiments, the passivation layer is an organic film disposed on the TFT layer to cover the TFT layer. In some embodiments, the planarization film is an organic film formed on the passivation layer. In some embodiments, the planarization film is formed of polyimide or acryl, like the organic film formed on the edge portion of the barrier layer. In some embodiments, the planarization film and the organic film are simultaneously formed when the OLED display is manufactured. In some embodiments, the organic film can be formed on the edge portion of the barrier layer such that a portion of the organic film directly contacts the base substrate and a remaining portion of the organic film contacts the barrier layer while surrounding the edge portion of the barrier layer.

In some embodiments, the light emitting layer includes a pixel electrode, a counter electrode, and an organic light emitting layer disposed between the pixel electrode and the counter electrode. In some embodiments, the pixel electrode is connected to the source/drain electrode of the TFT layer.

In some embodiments, when a voltage is applied to the pixel electrode through the TFT layer, an appropriate voltage is formed between the pixel electrode and the counter electrode, and thus the organic light emitting layer emits light, thereby forming an image. Hereinafter, an image forming unit including the TFT layer and the light emitting unit is referred to as a display unit.

In some embodiments, the encapsulation layer that covers the display unit and prevents penetration of external moisture can be formed to have a thin film encapsulation structure in which an organic film and an inorganic film are alternately stacked. In some embodiments, the encapsulation layer has a thin film encapsulation structure in which a plurality of thin films are stacked. In some embodiments, the organic film applied to the interface portion is spaced apart from each of the plurality of display units. In some embodiments, the organic film is formed such that a portion of the organic film directly contacts the base substrate and a remaining portion of the organic film contacts the barrier layer while surrounding an edge portion of the barrier layer.

In one embodiment, the OLED display is flexible and uses the soft base substrate formed of polyimide. In some embodiments, the base substrate is formed on a carrier substrate formed of a glass material, and then the carrier substrate is separated.

In some embodiments, the barrier layer is formed on a surface of the base substrate opposite to the carrier substrate. In one embodiment, the barrier layer is patterned according to a size of each of the cell panels. For example, while the base substrate is formed over the entire surface of a mother panel, the barrier layer is formed according to a size of each of the cell panels, and thus a groove is formed at an interface portion between the barrier layers of the cell panels. Each of the cell panels can be cut along the groove.

In some embodiments, the method of manufacture further comprises cutting along the interface portion, wherein a groove is formed in the barrier layer, wherein at least a portion of the organic film is formed in the groove, and wherein the groove does not penetrate into the base substrate. In some embodiments, the TFT layer of each of the cell panels is formed, and the passivation layer which is an inorganic film and the planarization film which is an organic film are disposed on the TFT layer to cover the TFT layer. At the same time as the planarization film formed of, for example, polyimide or acryl is formed, the groove at the interface portion is covered with the organic film formed of, for example, polyimide or acryl. This is to prevent cracks from occurring by allowing the organic film to absorb an impact generated when each of the cell panels is cut along the groove at the interface portion. That is, if the entire barrier layer is entirely exposed without the organic film, an impact generated when each of the cell panels is cut along the groove at the interface portion is transferred to the barrier layer, thereby increasing the risk of cracks. However, in one embodiment, since the groove at the interface portion between the barrier layers is covered with the organic film and the organic film absorbs an impact that would otherwise be transferred to the barrier layer, each of the cell panels can be softly cut and cracks can be prevented from occurring in the barrier layer. In one embodiment, the organic film covering the groove at the interface portion and the planarization film are spaced apart from each other. For example, if the organic film and the planarization film are connected to each other as one layer, since external moisture may penetrate into the display unit through the planarization film and a portion where the organic film remains, the organic film and the planarization film are spaced apart from each other such that the organic film is spaced apart from the display unit.

In some embodiments, the display unit is formed by forming the light emitting unit, and the encapsulation layer is disposed on the display unit to cover the display unit. As such, once the mother panel is completely manufactured, the carrier substrate that supports the base substrate is separated from the base substrate. In some embodiments, when a

laser beam is emitted toward the carrier substrate, the carrier substrate is separated from the base substrate due to a difference in a thermal expansion coefficient between the carrier substrate and the base substrate.

In some embodiments, the mother panel is cut in units of the cell panels. In some embodiments, the mother panel is cut along an interface portion between the cell panels by using a cutter. In some embodiments, since the groove at the interface portion along which the mother panel is cut is covered with the organic film, the organic film absorbs an impact during the cutting. In some embodiments, cracks can be prevented from occurring in the barrier layer during the cutting.

In some embodiments, the methods reduce a defect rate of a product and stabilize its quality.

Another aspect is an OLED display including: a barrier layer that is formed on a base substrate; a display unit that is formed on the barrier layer; an encapsulation layer that is formed on the display unit; and an organic film that is applied to an edge portion of the barrier layer.

<Method for Evaluating Delayed Fluorescent Material>

Next, a method for evaluating a delayed fluorescent material of the present invention will be described.

The method for evaluating a delayed fluorescent material of the invention includes evaluating the light emission characteristics of a delayed fluorescent material based on the ΔPBHT defined by the above-described formula (I). For the explanation of ΔPBHT, reference can be made to the description in the above-described section

<Organic Light Emitting Device>.

The delayed fluorescent material to be evaluated by the evaluation method of the invention is not particularly limited, and may be a known delayed fluorescent material or an unknown delayed fluorescent material. Furthermore, the delayed fluorescent material to be evaluated by the evaluation method of the invention may be a delayed fluorescent material having a ΔPBHT of 0.01 or more, or may be a delayed fluorescent material having a ΔPBHT of 0 or a delayed fluorescent material having a negative ΔPBHT. For the definition of the delayed fluorescent material and the explanation of the delayed fluorescent material having a ΔPBHT of 0.01 or more, reference can be made to the description in the above-described section [Delayed Fluorescent Material].

As will be shown in the Examples described below, there is a correlation between the ΔPBHT and the delayed fluorescence rate of a delayed fluorescent material. Therefore, when the ΔPBHT of the delayed fluorescent material as a target for evaluation is calculated, and the value of the delayed fluorescence rate corresponding to the ΔPBHT of the target for evaluation is determined from a correlation diagram of the ΔPBHT and the delayed fluorescence rate $\tau 2$ ($\tau 2$/PBHT correlation diagram), it can be predicted that the value is the delayed fluorescence rate of the target for evaluation. As a result, the delayed fluorescence rate of the target for evaluation and the light emission characteristics related to the delayed fluorescence rate can be evaluated. Examples of the light emission characteristics related to the delayed fluorescence rate include light emission efficiency, Foerster energy transfer efficiency, emission spectrum, half-value width, and device durability.

In an aspect of the present invention, the method for evaluating a delayed fluorescent material includes: determining the relationship between the ΔPBHT and the delayed fluorescence rate $\tau 2$ (for example, $\tau 2$/PBHT correlation diagram) based on the ΔPBHT and the delayed fluorescence rate of a plurality of kinds of reference delayed fluorescent materials having different ΔPBHT values; calculating the ΔPBHT of a delayed fluorescent material as a target for evaluation, determining the value of the delayed fluorescence rate corresponding to the ΔPBHT of the target for evaluation from the above-described relationship between the ΔPBHT and the delayed fluorescence rate $\tau 2$, and predicting this value as the delayed fluorescence rate of the target for evaluation; and evaluating the light emission characteristics of the target for evaluation based on the predicted delayed fluorescence rate.

Here, the delayed fluorescence rates of the reference delayed fluorescent materials may be measured values or calculated values; however, it is preferable that the delayed fluorescence rates are measured values that have been actually measured for the delayed fluorescent materials. Furthermore, regarding the relationship between the ΔPBHT and the delayed fluorescence rate $\tau 2$, the reference delayed fluorescent materials may be classified into groups each having a common ring skeleton, and the delayed fluorescence rate $\tau 2$ may be obtained for each group. In this case, the delayed fluorescence rate of a target for evaluation can be predicted with high accuracy by using the correlation obtained for a group of reference delayed fluorescent materials having a ring skeleton that is common or similar to that of the target for evaluation.

<Method for Designing Delayed Fluorescent Material>

A method for designing a delayed fluorescent material of the present invention includes performing molecular design of a delayed fluorescent material based on the relationship between the structure of a delayed fluorescent material and the ΔPBHT defined by the above-described formula (I). For the explanation and a preferred range of the ΔPBHT,

reference can be made to the description of the above-described section

<Organic Light Emitting Device>.

The delayed fluorescent material that is designed in the present invention may be a delayed fluorescent material having a ΔPBHT of 0.01 or more, or may be a delayed fluorescent material having a ΔPBHT of 0 or a delayed fluorescent material having a negative ΔPBHT. By designing the delayed fluorescent material so as to have a ΔPBHT of 0.01 or more, a delayed fluorescent material that has a high delayed fluorescence rate and is advantageous in improving the durability of an organic light emitting device can be obtained. For the definition of the delayed fluorescent material and the explanation and a preferred range of a delayed fluorescent material having a ΔPBHT of 0.01 or more, reference can be made to the description of the above-described section [Delayed Fluorescent Material].

The "relationship between the structure and the ΔPBHT of a delayed fluorescent material" in the present invention is a corresponding relationship between the structure and the ΔPBHT of a specific delayed fluorescent material, the corresponding relationship being determined based on the ΔPBHT values of the specific delayed fluorescent material and a plurality of kinds of compounds in which a part of the structure of the specific delayed fluorescent material has been changed. It is preferable that the partial change in the structure of the delayed fluorescent material is a change that can be quantified. Thereby, the "relationship between the structure and the ΔPBHT of a delayed fluorescent material" can be expressed as the relationship between the quantity and the ΔPBHT. Examples of the structural change that can be quantified include a change in the number of carbon atoms, a change in the number of substituents or deuterium atoms, a change in the substitution arrangement of substituents or deuterium atoms, a change in the number of fused rings, a change in the Hammett σp value of a substituent, and substitution of the number of atoms such as nitrogen, oxygen, and sulfur.

Furthermore, in the designing method of the invention, molecular design may be performed by taking into consideration the difference $\Delta E_{ST}$ between the lowest excited singlet energy and the lowest excited triplet energy. For example, by performing molecular design while also taking into consideration the relationship between the structure of the delayed fluorescent material and the $\Delta E_{ST}$, a delayed fluorescent material exhibiting desired light emission characteristics can be designed with high precision. Regarding the explanation and a preferred range of the $\Delta E_{ST}$, reference can be made to the description corresponding to the above-described section <Organic Light Emitting Device> and section [Delayed Fluorescent Material].

In an aspect of the present invention, the method for designing a delayed fluorescent material includes, as shown in FIG. 7: a first step (S 1) of calculating the ΔPBHT of a specific delayed fluorescent material; a second step (S2) of designing a modified compound in which a part of the structure of the specific delayed fluorescent material is changed, and calculating the ΔPBHT of the modified compound; a third step (S3) of designing a remodified compound in which a part of the structure of the modified compound is changed, and calculating the ΔPBHT of the remodified compound; a fourth step (S4) of determining the relationship between the compound structure and the ΔPBHT based on the structures of the specific delayed fluorescent material, the modified compound, and the remodified compound and the calculated ΔPBHT values; and a fifth step (S5) of extracting a compound structure corresponding to the ΔPBHT in a target range from the relationship between the compound structure and the ΔPBHT, and selecting a delayed fluorescent material to be synthesized from a group of compounds having the extracted structure. Here, the third step may be carried out repeatedly by regarding the remodified compound designed in this step as a modified compound.

In a preferred aspect of the invention, the designing method further includes determining the difference $\Delta E_{ST}$ between the lowest excited singlet energy and the lowest excited triplet energy for the specific delayed fluorescent material, the modified compound, and the remodified compound; and determining the relationship between the compound structure and $\Delta E_{ST}$ based on the structures of the specific delayed fluorescent material, the modified compound, and the remodified compound and the $\Delta E_{ST}$ values thereof, while in the fifth step, compound structures corresponding to the $\Delta E_{ST}$ in a target range are extracted from the relationship between the compound structure and the $\Delta E_{ST}$, and among those structures, one overlapping with the compound structure extracted from the relationship between the compound structure and the ΔPBHT is selected as the structure of a delayed fluorescent material to be synthesized.

In each of the above-described aspects of the present invention, the structures of the specific delayed fluorescent material used in the first step, the modified compound designed in the second step, and the remodified compound designed in the third step can be selected from structures represented by any of the above-described general formulae (1) to (6), but is not limited to these.

Furthermore, the first to the fifth steps may be carried out in this order, or may be carried out in a different order. For example, the calculation of the ΔPBHT in each step may be performed each time the compound is designed, in line with the compound design, or after the modified compound and remodified compound are designed, the ΔPBHT may be calculated collectively for the specific delayed fluorescent material, the modified compound, and the remodified compound.

<Method for Designing Organic Light Emitting Device>

A method for designing an organic light emitting device of the present invention includes selecting a delayed fluorescent material based on the ΔPBHT defined by the above-described formula (I) and designing an organic light emitting device using the selected delayed fluorescent material. For the explanation and a preferred range of the ΔPBHT, reference can be made to the description of the above-described section <Organic Light Emitting Device>. In the designing method of the invention, an organic light emitting device having high durability can be realized by selecting a delayed fluorescent material having a ΔPBHT of 0.01 or more and using the delayed fluorescent material for the design of an organic light emitting device. Regarding the definition of the delayed fluorescent material and the explanation and a preferred range of the delayed fluorescent material having a ΔPBHT of 0.01 or more, reference can be made to the description in the above-described section [Delayed Fluorescent Material]. For the configuration of the organic light emitting device, reference can be made to the description of the above-described section [Overall Configuration of Organic Light Emitting Device].

In an aspect of the present invention, the method for designing an organic light emitting device includes: searching for a delayed fluorescent material having a ΔPBHT of 0.01 or more from a database of delayed fluorescent materials storing the ΔPBHT of a plurality of kinds of delayed fluorescent materials as data; selecting a delayed fluorescent material to be used in an organic light emitting device from the group of delayed fluorescent materials found in the search; and designing an organic light emitting device using the selected delayed fluorescent material.

In a preferred aspect of the invention, the database of delayed fluorescent materials further stores the difference $\Delta E_{ST}$ between the lowest excited singlet energy and the lowest excited triplet energy of a plurality of delayed fluorescent materials as data, and in the above-described step of searching for a delayed fluorescent material, a delayed fluorescent material having a ΔPBHT of 0.01 or more and a $\Delta E_{ST}$ of 0.3 eV or less is searched for from the database of delayed fluorescent materials. For the explanation and a preferred range of the $\Delta E_{ST}$, reference can be made to corresponding descriptions in the above-described section <Organic Light Emitting Device> and section [Delayed Fluorescent Material].

<Program>

A program of the present invention is a program for carrying out at least one of the method for evaluating a delayed fluorescent material of the invention, the method for designing a delayed fluorescent material of the invention, and the method for designing an organic light emitting device of the invention.

For the step of configuring the program, reference can be made to the descriptions of each of the above-described sections <Method for Evaluating Delayed Fluorescent Material>, <Method for Designing Delayed Fluorescent Material>, and <Method for Designing Organic Light Emitting Device>.

The program of the invention may be a program for carrying out any one of the method for evaluating a delayed fluorescent material of the invention, the method for designing a delayed fluorescent material of the invention, and the method for designing an organic light emitting device of the invention, or may be a program for carrying out two or more of these methods. As an example of the latter, an aspect including a step of carrying out the method for evaluating a delayed fluorescent material of the invention and a step of carrying out the method for designing a delayed fluorescent material of the invention, in which a delayed fluorescent material designed by the method for designing a delayed fluorescent material of the invention is evaluated by the method for evaluating a delayed fluorescent material of the invention, may be mentioned.

Examples

The features of the present invention will be described more specifically with reference to Examples given below. The materials, processes, procedures, and the like shown below can be appropriately modified unless they deviate from the substance of the invention. Accordingly, the scope of the invention is not construed as being limited to the specific examples shown below. Incidentally, the light emission performance was evaluated using a source meter (available from Keithley Instruments: 2400 series), a semiconductor parameter analyzer (available from Agilent Technologies Japan, Ltd., E5273A), an optical power meter device (available from Newport Corporation, 1930C), an optical spectroscope (available from Ocean Optics Corporation, USB2000), a spectroradiometer (available from Topcon Corporation, SR-3), and a streak camera (available from HAMAMATSU PHOTONICS K.K., Model C4334), and the measurement of the delayed fluorescence rate was carried out using a Quantaurus-Tau small-sized fluorescence lifetime analyzer (available from HAMAMATSU PHOTONICS K.K., C16361-01).

(Example 1)

Production of Organic Electroluminescent Device Using Compound T1

On a glass substrate having a thickness of 2 mm, on which an anode having a film thickness of 50 nm and made of indium tin oxide (ITO) was formed, each thin film was laminated by a vacuum vapor deposition method at a degree of vacuum of $5.0 \times 10^{-5}$ Pa. First, HATCN was formed to a thickness of 10 nm on the ITO, and NPD was formed thereon to a thickness of 30 nm. Subsequently, TrisPCz was formed to a thickness of 10 nm, and PYD2Cz was formed thereon to a thickness of 5 nm. Next, the compound PYD2Cz and the compound T1 were co-deposited from different vapor deposition sources to form a light emitting layer having a thickness of 30 nm. At this time, the concentration of the compound T1 was 35% by weight. Next, SF3TRZ was formed to a thickness of 10 nm. Subsequently, SF3TRZ and Liq were co-deposited from different vapor deposition sources and were formed to a thickness of 30 nm. At this time, the concentration of Liq was 30% by weight. In addition, Liq was formed to a thickness of 2 nm, aluminum (Al) was vapor-deposited thereon to a thickness of 100 nm to form a cathode, and thus an organic electroluminescent device (EL Device 1) was obtained.

(Examples 2 to 12 and Comparative Examples 1 to 7) Production of Organic Electroluminescent Devices Using Compounds T2 to T12 or Comparative Compounds T1 to T7

Organic electroluminescent devices (EL Devices 2 to 12 and Comparative EL Devices 1 to 7) were produced in the same manner as in Example 1, except that the light emitting layer was formed using Compounds T2 to T12 or Comparative Compounds T1 to T7 instead of Compound T1.

For each of the produced EL devices, the delayed fluorescence rate (delay rate) and LT95 were measured, and the results of investigating the correlation between them and ΔPBHT are shown in FIG. 1 to FIG. 6. Here, LT95 is the time taken until the luminance reaches 95% of the initial luminance when continuously driven at 2.0 mA/$cm^2$. The Compounds T1 to T12 and Comparative Compounds T1 to T6 shown in FIG. 1 to FIG. 6 are delayed fluorescent materials contained in the light emitting layers of EL devices corresponding to the plots. The "ΔPBHT (T3-T1)" on the horizontal axis of each of FIGS. 1 and 3 represents ΔPBHT calculated from PBHT(T3) - PBHT(T1), and the "ΔPBHT(T2-T1)" on the horizontal axis of each of FIGS. 2 and 4 to 6 represents ΔPBHT calculated from PBHT(T2) - PBHT(T1).

As shown in FIGS. 1, 2, and 5, each of the EL devices that used the Compounds T1 to T12 having a ΔPBHT of 0.01 or more exhibited higher delayed fluorescence rates than each of the comparative EL devices that used the Comparative Compounds T1 to T7. Furthermore, as shown in FIGS. 3, 4, and 6, each of the EL devices that used the Compounds T1 to T12 had a much longer LT95 and a longer lifetime than each of the comparative EL devices that used the Comparative Compounds T1 to T7. Furthermore, a correlation was observed in which as the ΔPBHT of the delayed fluorescent material was larger, the delayed fluorescence rate and LT95 increased.

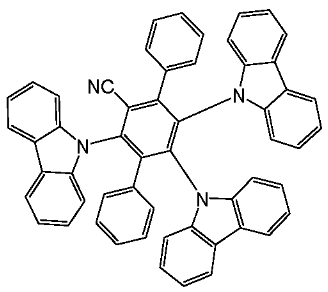

Comparative Compound T1

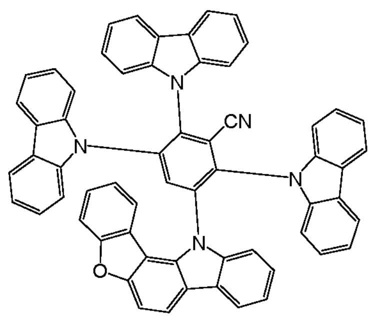

Comparative Compound T2

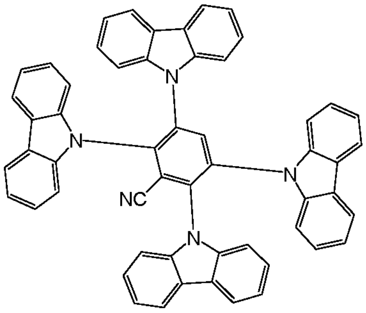

Comparative Compound T3

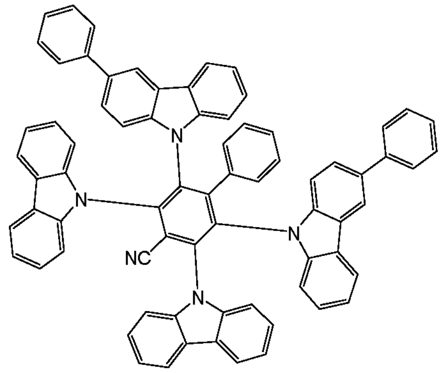

Comparative Compound T4

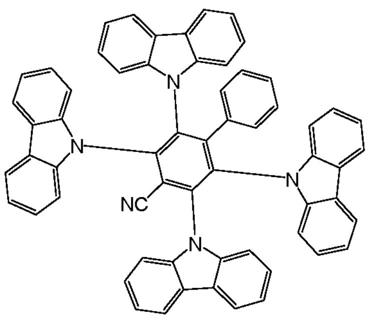

Comparative Compound T5

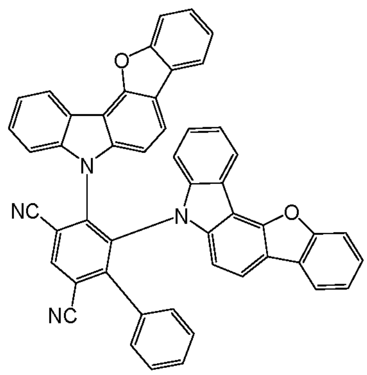

Comparative Compound T6

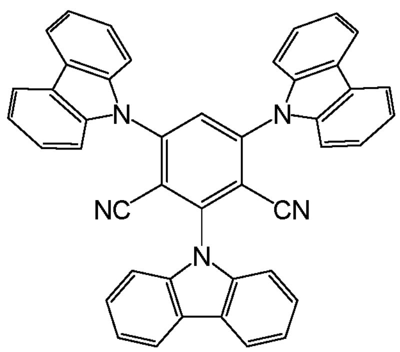

Comparative Compound T7

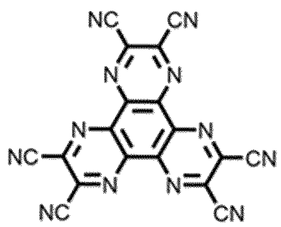

HATCN

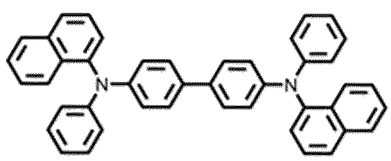

NPD

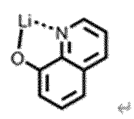

Liq

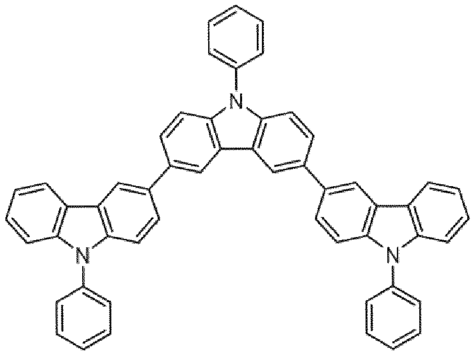

TrisPCz

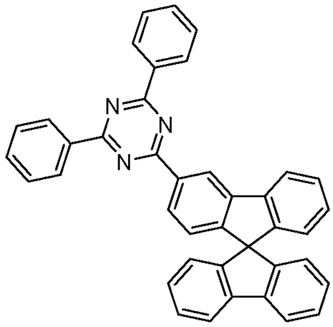

SF3TRZ

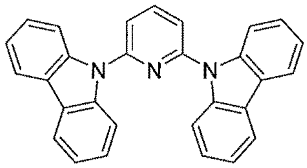

PYD2Cz

Industrial Applicability

In the present invention, durability of an organic light emitting device can be improved by using a delayed fluorescent material having a ΔPBHT of 0.01 or more. Therefore, according to the present invention, a delayed fluorescent type organic light emitting device having excellent practical usability can be provided. Thus, the present invention has high industrial applicability.

**Claims**

1. An organic light emitting device comprising:

a light emitting layer containing a delayed fluorescent material having a ΔPBHT of 0.01 or more as defined by the following formula (I):

$$\text{Formula (I)} \qquad \Delta PBHT = PBHT(Tn) - PBHT(T1)$$

wherein in formula (I), PBHT(T1) represents a PBHT value of a lowest excited triplet state of the delayed fluorescent material; and PBHT(Tn) represents a PBHT value of an excited triplet state having a smallest energy among excited triplet states having larger energies than a lowest excited singlet energy of the delayed fluorescent material.

2. The organic light emitting device according to claim 1, wherein the delayed fluorescent material is a compound represented by any of the following general formulae (1) to (6):

General Formula (1)

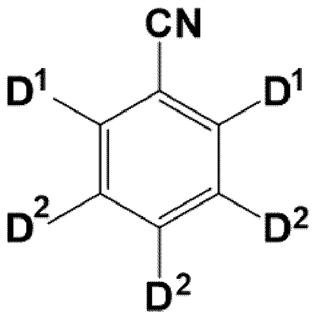

General Formula (2)

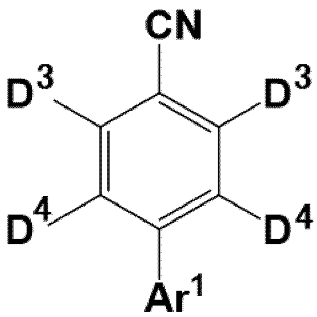

General Formula (3)

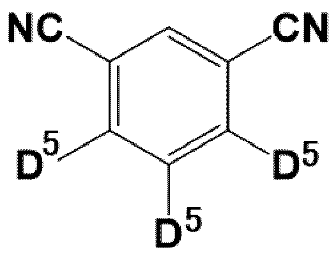

General Formula (4)

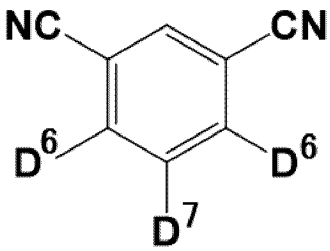

General Formula (5)

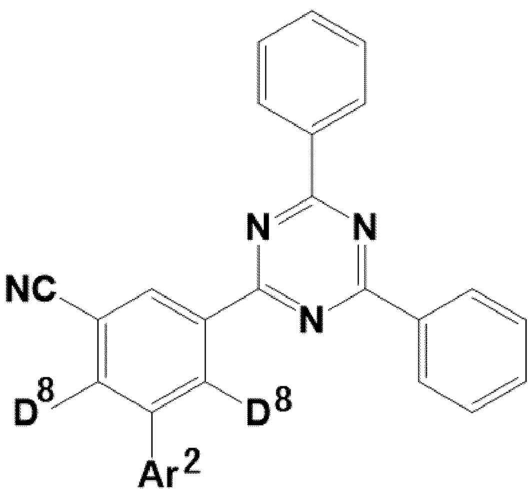

General Formula (6)

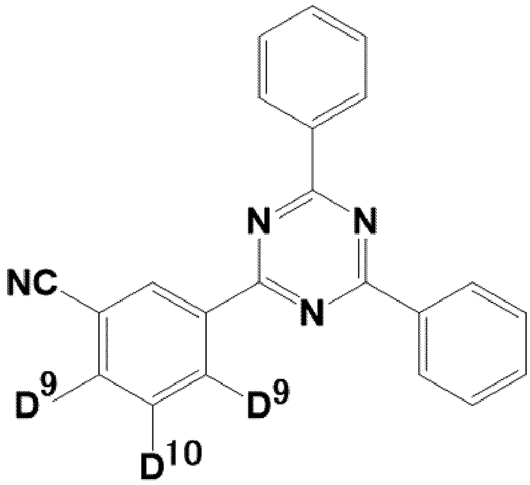

wherein in general formulae (1) to (6), $D^1$ to $D^{10}$ each independently represent a group represented by the following general formula (7), provided that $D^1$ and $D^2$, $D^3$ and $D^4$, $D^6$ and $D^7$, and $D^9$ and $D^{10}$ have chemical structures different from each other, and two $D^1$'s, three $D^2$'s, two $D^3$'s, two $D^4$'s, three $D^5$'s, two $D^6$'s, two $D^8$'s, and two $D^9$'s have chemical structures identical with each other;

General Formula (7)

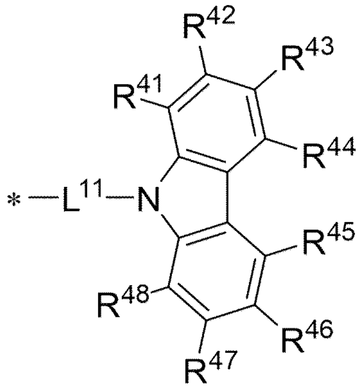

wherein in general formula (7), $L^{11}$ represents a single bond or a divalent linking group; $R^{41}$ to $R^{48}$ each independently represent a hydrogen atom or a substituent; $R^{41}$ and $R^{42}$, $R^{42}$ and $R^{43}$, $R^{43}$ and $R^{44}$, $R^{44}$ and $R^{45}$, $R^{45}$ and $R^{46}$, $R^{46}$ and $R^{47}$, and $R^{47}$ and $R^{48}$ may be bonded to each other to form a cyclic structure.

**3.** The organic light emitting device according to claim 2, wherein in each of the general formulae (1) to (6), at least one of the groups represented by the general formula (7) is a group represented by any of the following general formulae (8) to (13):

General Formula (8)

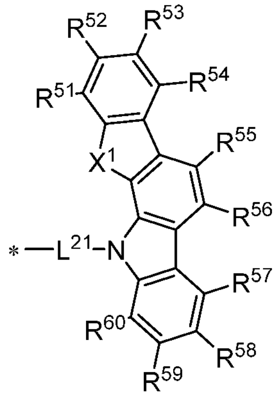

General Formula (9)

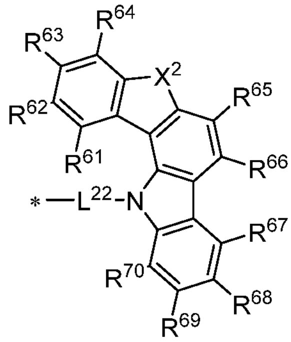

General Formula (10)

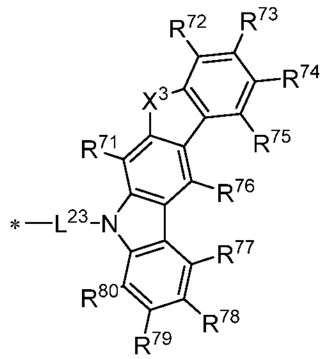

General Formula (11)

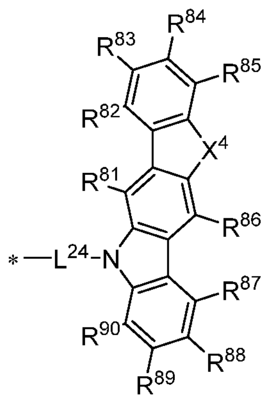

General Formula (12)

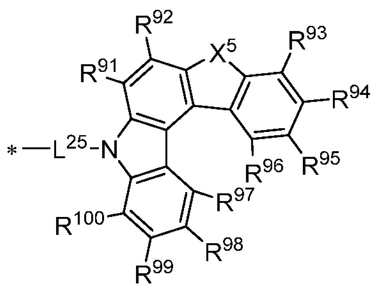

General Formula (13)

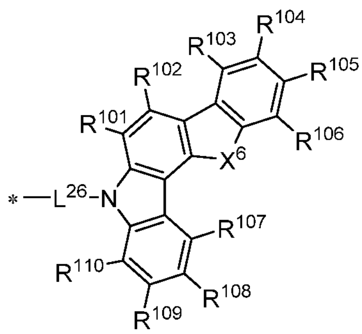

wherein in general formulae (8) to (13), $L^{21}$ to $L^{26}$ each represent a single bond or a divalent linking group; $R^{51}$ to $R^{110}$ each independently represent a hydrogen atom or a substituent; and $R^{51}$ and $R^{52}$, $R^{52}$ and $R^{53}$, $R^{53}$ and $R^{54}$, $R^{54}$ and $R^{55}$, $R^{55}$ and $R^{56}$, $R^{56}$ and $R^{57}$, $R^{57}$ and $R^{58}$ , $R^{58}$ and $R^{59}$, $R^{59}$ and $R^{60}$, $R^{61}$ and R $^{62}$, $R^{62}$ and $R^{63}$, $R^{63}$ and R6$^{4}$ , $R^{65}$ and $R^{66}$, $R^{66}$ and $R^{67}$, $R^{67}$ and $R^{68}$, $R^{68}$ and $R^{69}$, $R^{69}$ and $R^{70}$, $R^{72}$ and $R^{73}$, $R^{73}$ and $R^{74}$, $R^{74}$ and $R^{75}$, $R^{75}$ and $R^{76}$, $R^{76}$ and $R^{77}$, $R^{77}$ and $R^{78}$, $R^{78}$ and $R^{79}$, $R^{79}$ and $R^{80}$, $R^{81}$ and $R^{82}$, $R^{82}$ and $R^{83}$, $R^{83}$ and $R^{84}$, $R^{84}$ and $R^{85}$, $R^{86}$ and $R^{87}$, $R^{87}$ and $R^{88}$, $R^{88}$ and $R^{89}$, $R^{89}$ and $R^{90}$, $R^{91}$ and $R^{92}$, $R^{93}$ and $R^{94}$, $R^{94}$ and $R^{95}$, $R^{95}$ and $R^{96}$, $R^{96}$ and $R^{97}$, $R^{97}$ and $R^{98}$, $R^{99}$ and $R^{100}$, $R^{101}$ and $R^{102}$, $R^{102}$ and $R^{103}$, $R^{103}$ and $R^{104}$, $R^{104}$ and $R^{105}$, $R^{105}$ and $R^{106}$, $R^{107}$ and $R^{108}$, $R^{108}$ and $R^{109}$, and $R^{109}$ and $R^{110}$ may be bonded to each other to form a cyclic structure.

4. The organic light emitting device according to any one of claims 1 to 3, wherein the PBHT(Tn) is PBHT(T3) of a third excited triplet state.

5. The organic light emitting device according to claim 4, wherein the delayed fluorescent material is a compound

represented by the general formula (2).

**6.** The organic light emitting device according to claim 5, wherein $D^4$ in the general formula (2) is a group represented by the general formula (13).

**7.** The organic light emitting device according to any one of claims 1 to 3, wherein the PBHT(Tn) is PBHT(T2) of a second excited triplet state.

**8.** The organic light emitting device according to claim 7, wherein the delayed fluorescent material is a compound represented by the general formula (4) or (5).

**9.** The organic light emitting device according to claim 8, wherein $D^6$ in the general formula (4) and $D^8$ in the general formula (5) are each a group represented by the general formula (13).

**10.** A method for evaluating a delayed fluorescent material, the method comprising:

evaluating light emission characteristics of a delayed fluorescent material based on ΔPBHT defined by the following formula (I):

$$\text{Formula (I)} \qquad \Delta PBHT = PBHT(Tn) - PBHT(T1)$$

wherein in formula (I), PBHT(T1) represents a PBHT value of a lowest excited triplet state of the delayed fluorescent material; and PBHT(Tn) represents a PBHT value of an excited triplet state having a smallest energy among excited triplet states having larger energies than a lowest excited singlet energy of the delayed fluorescent material.

**11.** The evaluation method according to claim 10, comprising:
predicting a delayed fluorescence rate of the delayed fluorescent material based on the ΔPBHT.

**12.** The evaluation method according to claim 10, comprising:

determining a relationship between ΔPBHT and a delayed fluorescence rate $\tau 2$ based on ΔPBHT values and delayed fluorescence rates of a plurality of kinds of reference delayed fluorescent materials having different ΔPBHT values;
calculating ΔPBHT of a delayed fluorescent material as a target for evaluation, determining a value of the delayed fluorescence rate corresponding to the ΔPBHT of the target for evaluation from the relationship between the ΔPBHT and the delayed fluorescence rate $\tau 2$, and predicting this value to be the delayed fluorescence rate of the target for evaluation; and
evaluating light emission characteristics of the target for evaluation based on the predicted delayed fluorescence rate.

**13.** The evaluation method according to claim 12, wherein the delayed fluorescence rates of the reference delayed fluorescent materials are measured values.

**14.** A method for designing a delayed fluorescent material, the method comprising:

performing molecular design of a delayed fluorescent material based on a relationship between a structure of the delayed fluorescent material and ΔPBHT defined by the following formula (I):

$$\text{Formula (I)} \qquad \Delta PBHT = PBHT(Tn) - PBHT(T1)$$

wherein in formula (I), PBHT(T1) represents a PBHT value of a lowest excited triplet state of the delayed fluorescent material; and PBHT(Tn) represents a PBHT value of an excited triplet state having a smallest energy among excited triplet states having larger energies than a lowest excited singlet energy of the delayed fluorescent material.

**15.** The designing method according to claim 14, comprising:

a first step of calculating ΔPBHT of a specific delayed fluorescent material;
a second step of designing a modified compound in which a part of a structure of the specific delayed fluorescent material is changed, and calculating ΔPBHT of the modified compound;
a third step of designing a remodified compound in which a part of a structure of the modified compound is changed, and calculating ΔPBHT of the remodified compound;
a fourth step of determining a relationship between a compound structure and ΔPBHT based on structures of the specific delayed fluorescent material, the modified compound, and the remodified compound and the calculated ΔPBHT values; and
a fifth step of extracting a compound structure corresponding to ΔPBHT in a target range from the relationship between a compound structure and ΔPBHT, and selecting a delayed fluorescent material to be synthesized from a group of compounds having the extracted structure.

**16.** The designing method according to claim 15, wherein a partial change in the structures of the specific delayed fluorescent material and the modified compound is a quantifiable change.

**17.** The designing method according to claim 15, further comprising:

determining a difference $\Delta E_{ST}$ between a lowest excited singlet energy and a lowest excited triplet energy for the specific delayed fluorescent material, the modified compound, and the remodified compound,
wherein in the fifth step, a delayed fluorescent material to be synthesized is selected from the group of compounds, based on the $\Delta E_{ST}$ determined in the above-described step.

**18.** The designing method according to claim 15, wherein the remodified compound designed in the third step is regarded as a modified compound, and the third step is repeatedly carried out.

**19.** A method for designing an organic light emitting device, the method comprising:

selecting a delayed fluorescent material based on ΔPBHT defined by the following formula (I), and designing an organic light emitting device using the selected delayed fluorescent material:

$$\text{Formula (I)} \qquad \Delta PBHT = PBHT(Tn) - PBHT(T1)$$

wherein in formula (I), PBHT(T1) represents a PBHT value of a lowest excited triplet state of the delayed fluorescent material; and PBHT(Tn) represents a PBHT value of an excited triplet state having a smallest energy among excited triplet states having larger energies than a lowest excited singlet energy of the delayed fluorescent material.

**20.** The designing method according to claim 19, comprising:

searching for a delayed fluorescent material having a ΔPBHT of 0.01 or more from a database of delayed fluorescent materials storing ΔPBHT values of a plurality of kinds of delayed fluorescent materials as data;
selecting a delayed fluorescent material to be used in an organic light emitting device, from a group of delayed fluorescent materials found in the search in the above-described step; and
designing an organic light emitting device using the delayed fluorescent material selected in the above-described step.

**21.** The designing method according to claim 20, wherein the database of delayed fluorescent materials further stores differences $\Delta E_{ST}$ between a lowest excited singlet energy and a lowest excited triplet energy of the plurality of delayed fluorescent materials as data, and in the step of searching for a delayed fluorescent material, a delayed fluorescent material having a ΔPBHT of 0.01 or more and a $\Delta E_{ST}$ of 0.3 eV or less is searched for from the database of delayed fluorescent materials.

**22.** A program for carrying out the method according to any one of claims 10 to 21.

FIG. 1

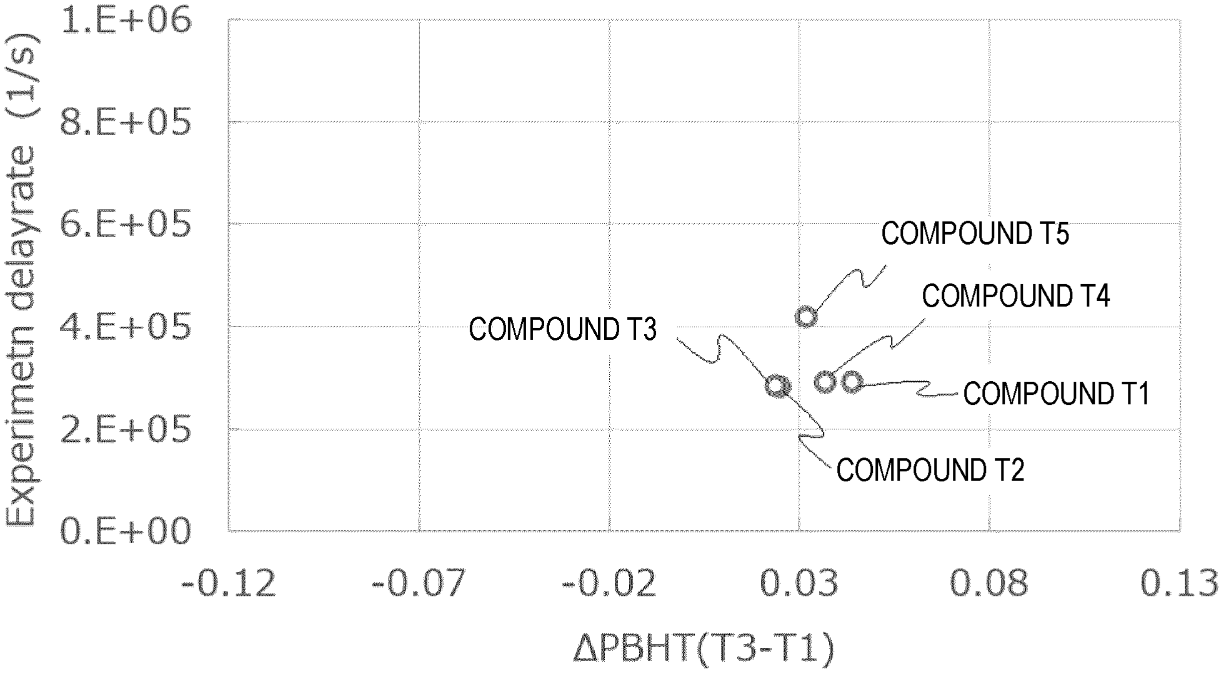
1.E+06
8.E+05
6.E+05
4.E+05
2.E+05
0.E+00
Experimetn delayrate (1/s)
-0.12
-0.07
-0.02
0.03
0.08
0.13
ΔPBHT(T3-T1)
COMPOUND T5
COMPOUND T4
COMPOUND T3
COMPOUND T1
COMPOUND T2

FIG. 2

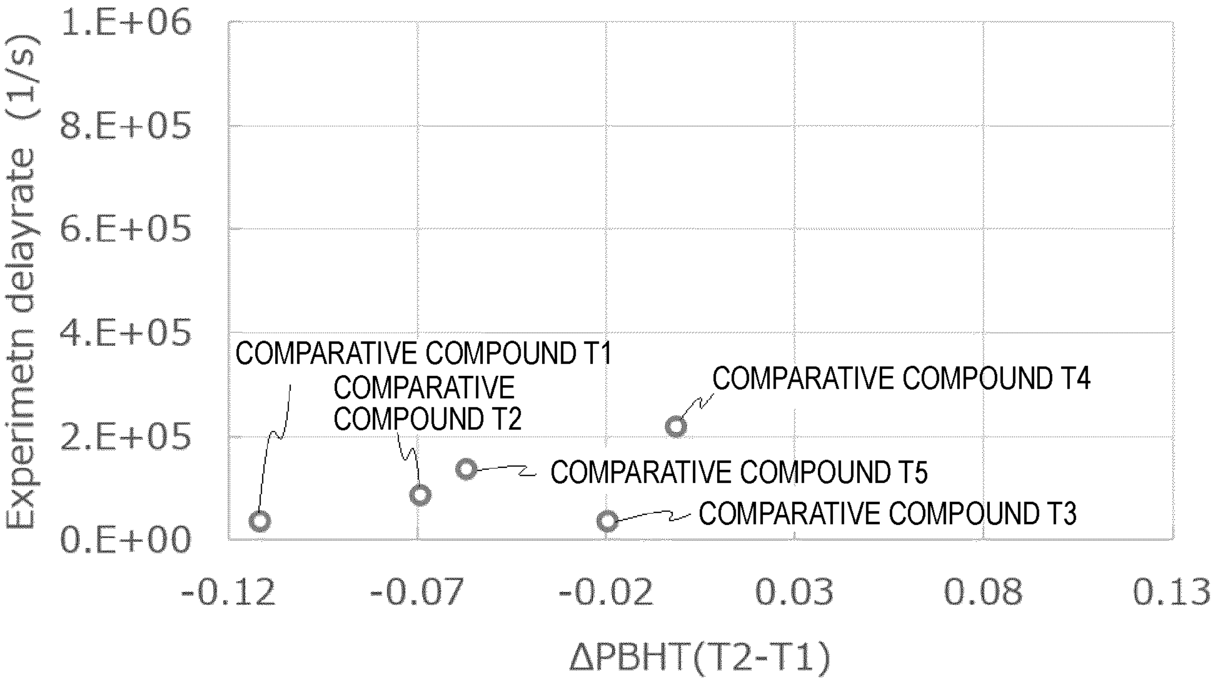
1.E+06
8.E+05
6.E+05
4.E+05
2.E+05
0.E+00
Experimetn delayrate (1/s)
-0.12
-0.07
-0.02
0.03
0.08
0.13
ΔPBHT(T2-T1)
COMPARATIVE COMPOUND T1
COMPARATIVE COMPOUND T2
COMPARATIVE COMPOUND T4
COMPARATIVE COMPOUND T5
COMPARATIVE COMPOUND T3

FIG. 3

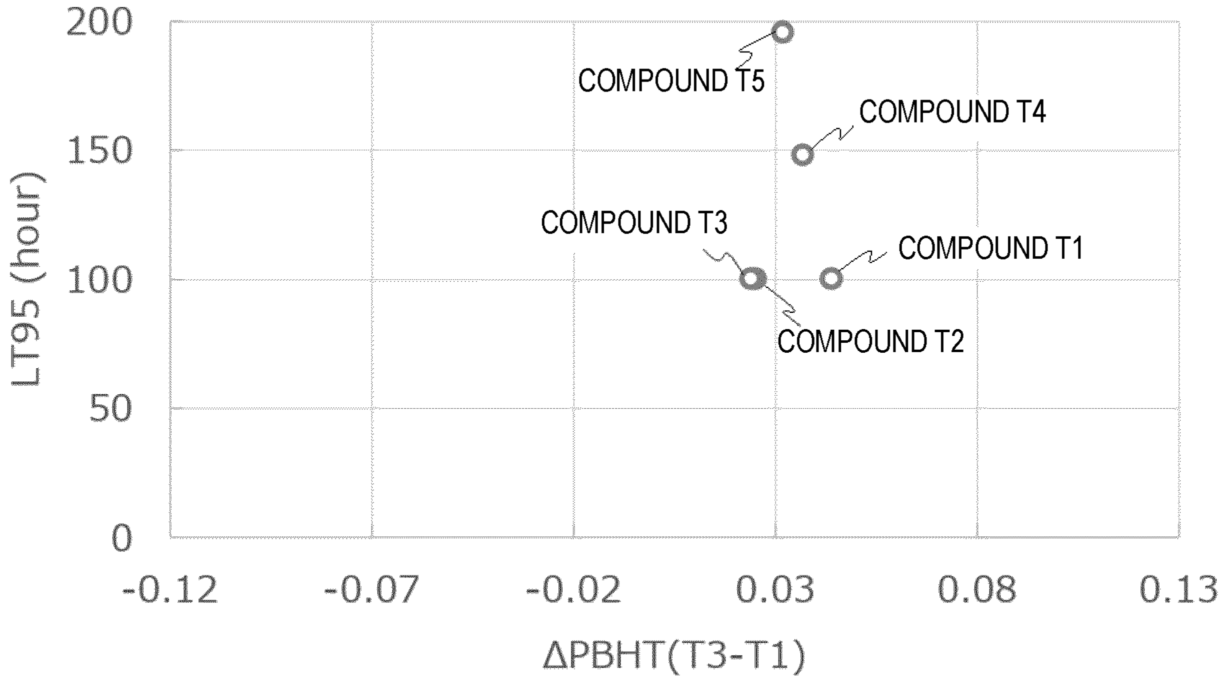
200
150
100
50
0
LT95 (hour)
-0.12
-0.07
-0.02
0.03
0.08
0.13
ΔPBHT(T3-T1)
COMPOUND T5
COMPOUND T4
COMPOUND T3
COMPOUND T1
COMPOUND T2

FIG. 4

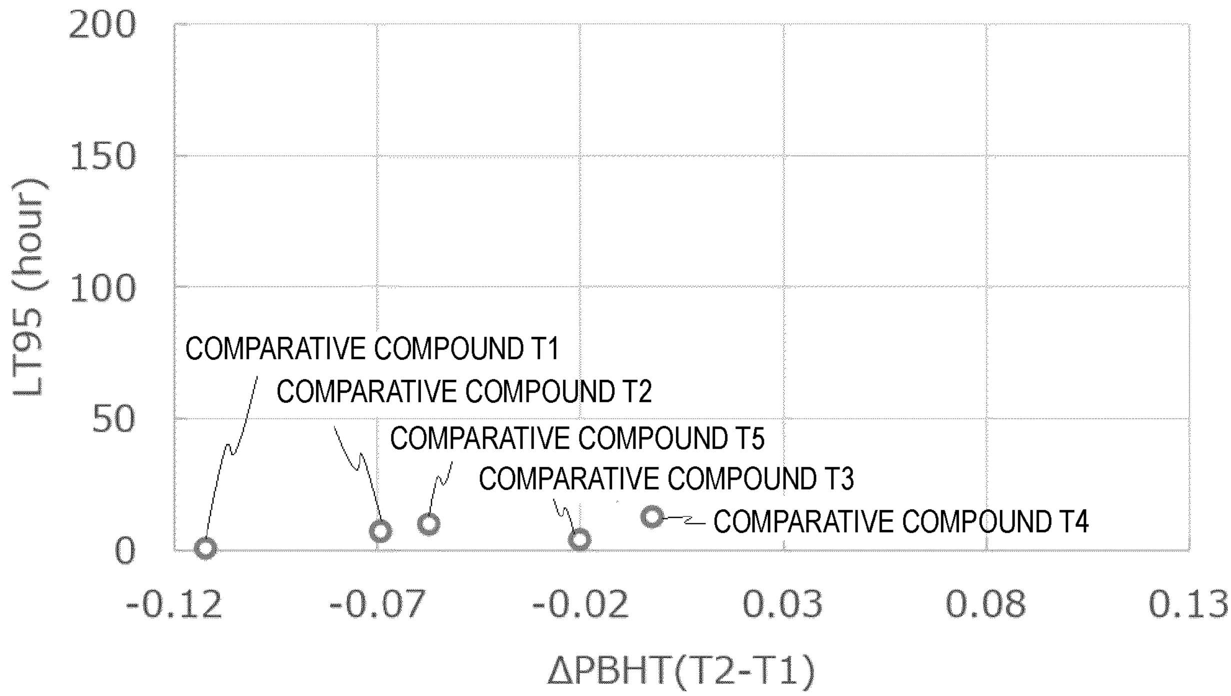
200
150
100
50
0
LT95 (hour)
-0.12
-0.07
-0.02
0.03
0.08
0.13
ΔPBHT(T2-T1)
COMPARATIVE COMPOUND T1
COMPARATIVE COMPOUND T2
COMPARATIVE COMPOUND T5
COMPARATIVE COMPOUND T3
COMPARATIVE COMPOUND T4

FIG. 5

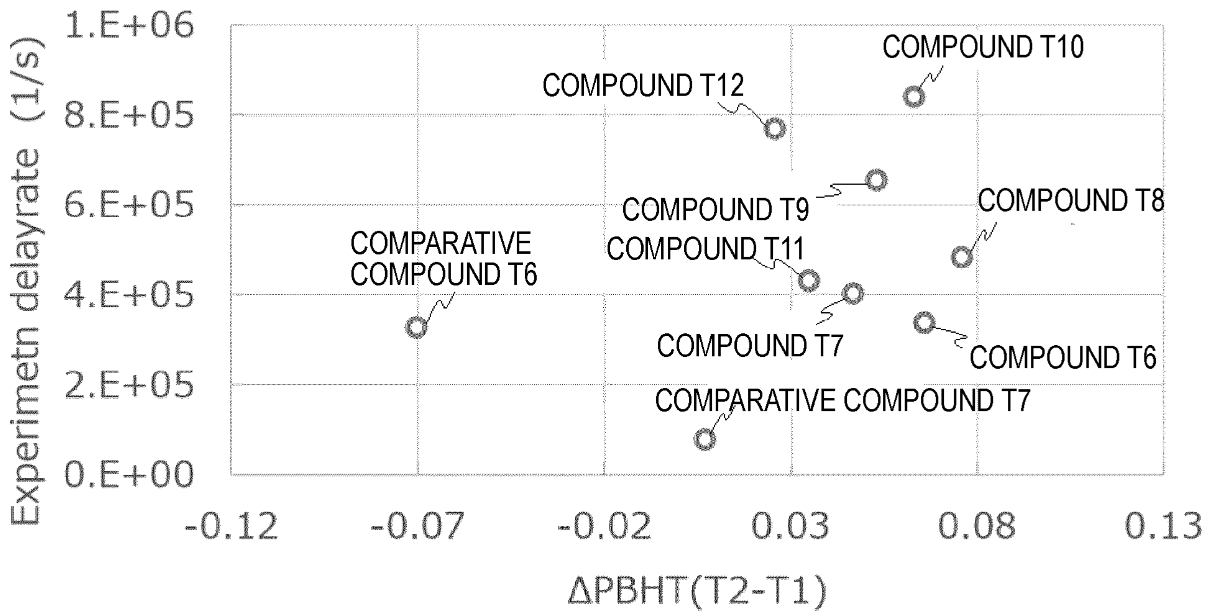
1.E+06
8.E+05
6.E+05
4.E+05
2.E+05
0.E+00
Experimetn delayrate (1/s)
-0.12
-0.07
-0.02
0.03
0.08
0.13
ΔPBHT(T2-T1)
COMPOUND T10
COMPOUND T12
COMPOUND T9
COMPOUND T8
COMPARATIVE COMPOUND T6
COMPOUND T11
COMPOUND T7
COMPOUND T6
COMPARATIVE COMPOUND T7

FIG. 6

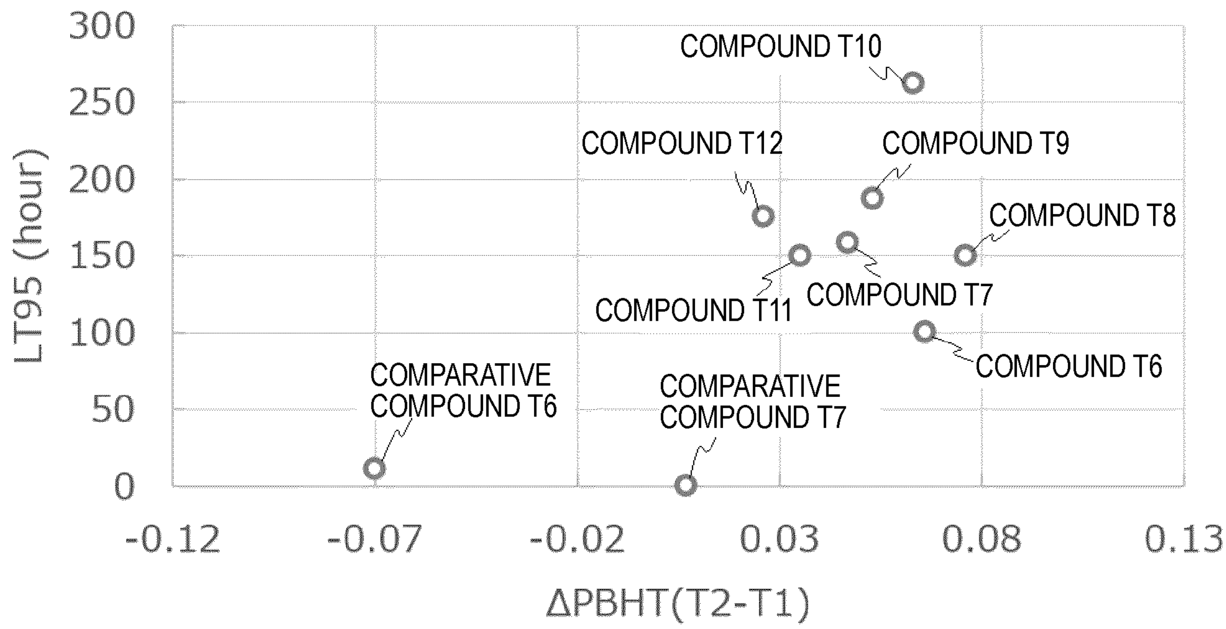
300
250
200
150
100
50
0
LT95 (hour)
-0.12
-0.07
-0.02
0.03
0.08
0.13
ΔPBHT(T2-T1)
COMPOUND T10
COMPOUND T12
COMPOUND T9
COMPOUND T8
COMPOUND T11
COMPOUND T7
COMPOUND T6
COMPARATIVE COMPOUND T6
COMPARATIVE COMPOUND T7

FIG. 7

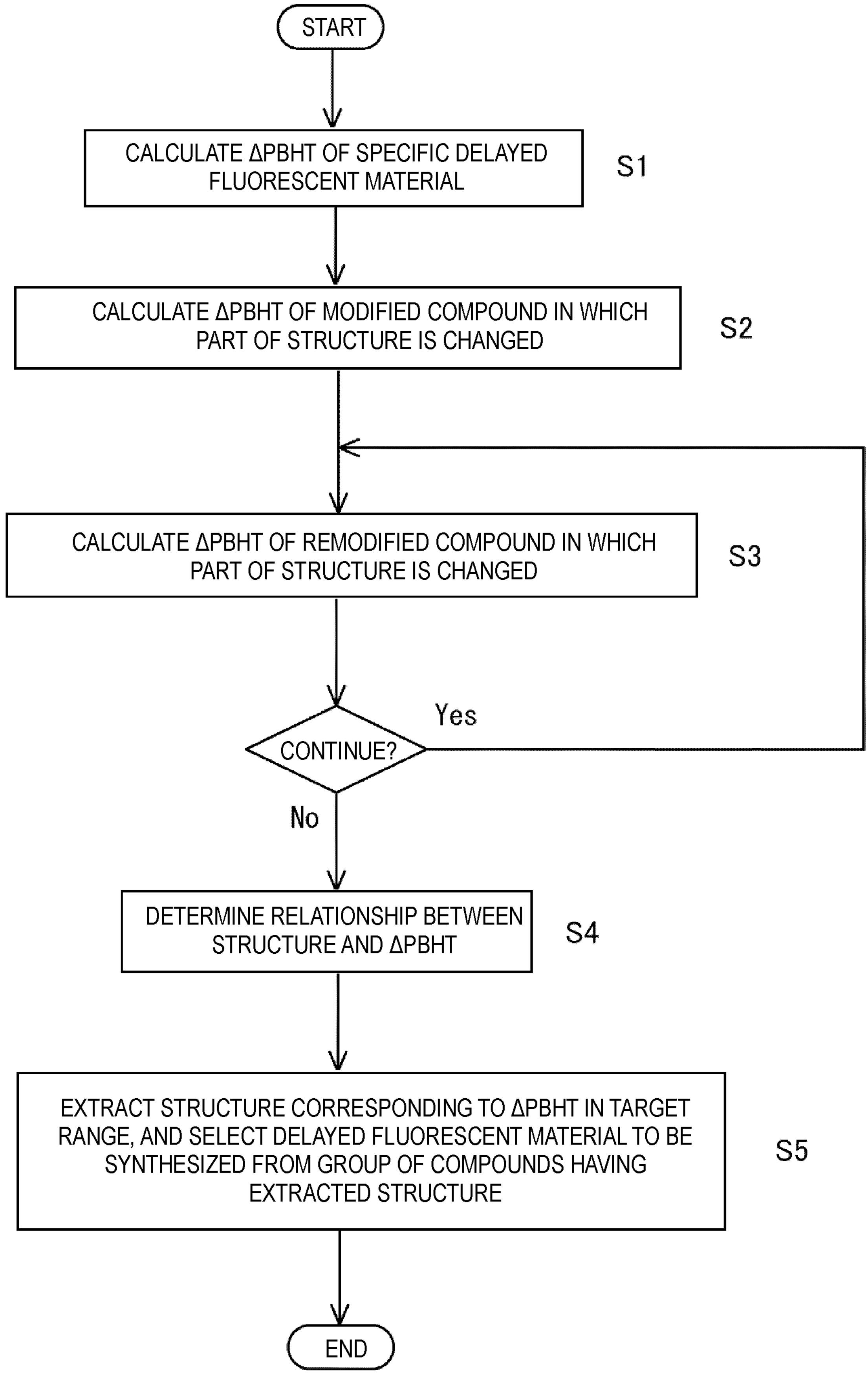
START
CALCULATE ΔPBHT OF SPECIFIC DELAYED FLUORESCENT MATERIAL
S1
CALCULATE ΔPBHT OF MODIFIED COMPOUND IN WHICH PART OF STRUCTURE IS CHANGED
S2
CALCULATE ΔPBHT OF REMODIFIED COMPOUND IN WHICH PART OF STRUCTURE IS CHANGED
S3
Yes
CONTINUE?
No
DETERMINE RELATIONSHIP BETWEEN STRUCTURE AND ΔPBHT
S4
EXTRACT STRUCTURE CORRESPONDING TO ΔPBHT IN TARGET RANGE, AND SELECT DELAYED FLUORESCENT MATERIAL TO BE SYNTHESIZED FROM GROUP OF COMPOUNDS HAVING EXTRACTED STRUCTURE
S5
END

**INTERNATIONAL SEARCH REPORT**

International application No.
**PCT/JP2023/007738**

**A. CLASSIFICATION OF SUBJECT MATTER**

***H10K 85/60***(2023.01)i; ***C09K 11/06***(2006.01)i; ***H10K 50/12***(2023.01)i; ***H10K 71/70***(2023.01)i; *H10K 101/20*(2023.01)n; *H10K 101/30*(2023.01)n
FI: H10K85/60; C09K11/06 645; C09K11/06 655; H10K50/12; H10K71/70; H10K101:20; H10K101:30

According to International Patent Classification (IPC) or to both national classification and IPC

**B. FIELDS SEARCHED**

Minimum documentation searched (classification system followed by classification symbols)

H10K85/60; C09K11/06; H10K50/12; H10K71/70; H10K101/20; H10K101/30

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2022/025248 A1 (KYULUX INC.) 03 February 2022 (2022-02-03)<br>claims, paragraph [0044], compound 570243, paragraph [0107], compound 570261, paragraph [0121] | 1-3, 7-9<br>4-6, 10-22 |
| X<br>A | WO 2021/241677 A1 (KYULUX INC.) 02 December 2021 (2021-12-02)<br>claims, paragraphs [0022], [0024]-[0053], [0095]-[0099], examples | 1-9<br>10-22 |
| P, A | CN 114685355 A (BEIJING TRIPOD MATERIAL SCIENCE AND TECHNOLOGY LIMITED COMPANY) 01 July 2022 (2022-07-01)<br>entire text | 1-22 |
| P, A | JP 2022-129192 A (KYUSHU UNIVRSITY, NATIONAL UNIVERSITY CORP.) 05 September 2022 (2022-09-05)<br>entire text, all drawings | 1, 10-22 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 April 2023** | **18 April 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/007738**

**C. DOCUMENTS CONSIDERED TO BE RELEVANT**

| Category* | Citation of document, with indication, where appropriate, of the relevant passages | Relevant to claim No. |
|---|---|---|
| P, A | JP 2023-7515 A (KYOTO UNIVERSITY) 19 January 2023 (2023-01-19) fig. 8-10, paragraphs [0034]-[0038] | 1, 10-22 |

Form PCT/ISA/210 (second sheet) (January 2015)

**INTERNATIONAL SEARCH REPORT**
**Information on patent family members**

International application No.
**PCT/JP2023/007738**

| Patent document cited in search report | Publication date (day/month/year) | Patent family member(s) | Publication date (day/month/year) |
|---|---|---|---|
| WO 2022/025248 A1 | 03 February 2022 | JP 2022-27733 A | |
| | | TW 202216956 A | |
| WO 2021/241677 A1 | 02 December 2021 | WO 2022/249505 A1 | |
| | | WO 2022/249506 A1 | |
| | | TW 202204319 A | |
| CN 114685355 A | 01 July 2022 | (Family: none) | |
| JP 2022-129192 A | 05 September 2022 | (Family: none) | |
| JP 2023-7515 A | 19 January 2023 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *J. Chem. Phys.*, 2008, vol. 128, 044118
- Excitation energies in density functional theory: An evaluation and a diagnostic test. *J. Chem. Phys.*, 2008, vol. 128, 044118
- **HANSCH, C.** *Chem. Rev.*, 1991, vol. 91, 165-195